# EUROPEAN PATENT SPECIFICATION

(11) **EP 3 027 771 B1**
(45) Date of publication and mention of the grant of the patent: **16.01.2019**
(21) Application number: 14831238.2
(22) Date of filing: 30.07.2014
(51) Int. Cl.: C12Q 1/68, C12N 15/10

(54) **METHODS FOR THE PRODUCTION OF LONG LENGTH CLONAL SEQUENCE VERIFIED NUCLEIC ACID CONSTRUCTS**
VERFAHREN ZUR HERSTELLUNG VON LANGEN KLONALEN SEQUENZVERIFIZIERTEN NUKLEINSÄUREKONSTRUKTEN
PROCÉDÉS POUR LA PRODUCTION DE CONSTRUCTIONS D'ACIDE NUCLÉIQUE VÉRIFIÉES PAR UNE SÉQUENCE CLONALE DE GRANDE LONGUEUR

(30) Priority: 30.07.2013 US 201361859946 P; 27.11.2013 US 201361909526 P
(43) Date of publication of application: 08.06.2016
(73) Proprietor: Gen9, Inc., Cambridge, MA 02139 (US)
(72) Inventor: JACOBSON, Joseph, Newton Massachusetts 02459 (US); HUDSON, Michael E., Framingham Massachusetts 01701 (US); KUNG, Li-yun, Arlington Massachusetts 02474 (US)
(74) Representative: Sutcliffe, Nicholas Robert
(86) International application number: PCT/US2014/048867
(87) International publication number: WO 2015/017527

(56) References cited:
- WO-A1-2011/014811
- WO-A2-2013/163263
- US-A1- 2011 287 435
- US-A1- 2012 208 705
- US-A1- 2013 017 978
- US-B1- 6 881 539
- LIU, L ET AL.: 'Comparison Of Next-Generation Sequencing Systems.' JOURNAL OF BIOMEDICINE AND BIOTECHNOLOGY. vol. 2012, 02 April 2012, page 2, XP055232530

## Description

### RELATED APPLICATIONS

### FIELD OF THE INVENTION

Methods and compositions of the invention relate to nucleic acid assembly, and particularly to methods for sorting and cloning nucleic acids having a predetermined sequence.

### BACKGROUND

Recombinant and synthetic nucleic acids have many applications in research, industry, agriculture, and medicine. Recombinant and synthetic nucleic acids can be used to express and obtain large amounts of polypeptides, including enzymes, antibodies, growth factors, receptors, and other polypeptides that may be used for a variety of medical, industrial, or agricultural purposes. Recombinant and synthetic nucleic acids also can be used to produce genetically modified organisms including modified bacteria, yeast, mammals, plants, and other organisms. Genetically modified organisms may be used in research (e.g., as animal models of disease, as tools for understanding biological processes, etc.), in industry (e.g., as host organisms for protein expression, as bioreactors for generating industrial products, as tools for environmental remediation, for isolating or modifying natural compounds with industrial applications, etc.), in agriculture (e.g., modified crops with increased yield or increased resistance to disease or environmental stress, etc.), and for other applications. Recombinant and synthetic nucleic acids also may be used as therapeutic compositions (e.g., for modifying gene expression, for gene therapy, etc.) or as diagnostic tools (e.g., as probes for disease conditions, etc.).

Numerous techniques have been developed for modifying existing nucleic acids (e.g., naturally occurring nucleic acids) to generate recombinant nucleic acids. For example, combinations of nucleic acid amplification, mutagenesis, nuclease digestion, ligation, cloning and other techniques may be used to produce many different recombinant nucleic acids. Chemically synthesized polynucleotides are often used as primers or adaptors for nucleic acid amplification, mutagenesis, and cloning.

Techniques also are being developed for de novo nucleic acid assembly whereby nucleic acids are made (e.g., chemically synthesized) and assembled to produce longer target nucleic acids of interest. For example, different multiplex assembly techniques are being developed for assembling oligonucleotides into larger synthetic nucleic acids that can be used in research, industry, agriculture, and/or medicine. However, one limitation of currently available assembly techniques is the relatively high error rate. As such, low cost production methods of long length high fidelity nucleic acids are needed.

### SUMMARY OF THE INVENTION

Aspects of the invention relate to methods for preparing nucleic acid molecules as set out in the claims. In some disclosure herein methods and compositions for the production of nucleic acid molecules having a length of 1kbase or more are provided.

In some disclosure herein, the methods comprise providing a pool of nucleic acid molecules comprising at least two populations of nucleic acid molecules, each population of nucleic acid molecule having at least one unique target nucleic acid sequence, the target nucleic acid sequence having an oligonucleotide tag sequence at its 5' end and at its 3' end. The oligonucleotide tag sequence can comprise a unique nucleotide tag. The nucleic acid molecules can be subjected to fragmentation to generate nucleic acid fragments, wherein the nucleic acid fragments comprise oligonucleotide tag sequences at their 5' end and at their 3' end, the oligonucleotide tag sequence comprising a unique nucleotide tag. The sequence of the tagged nucleic acid fragments can be determined.

In some disclosure herein the pool of nucleic acid molecules comprises error-free and error-containing nucleic acid molecules. In some disclosure herein the method further comprises isolating the nucleic acid molecules having the predetermined sequence.

In some disclosure herein a pool of nucleic acid molecules comprising at least two populations of nucleic acid molecules is provided. Each population of nucleic acid molecules can have a unique target nucleic acid sequence, the target nucleic acid sequence having a 5' end and a 3' end. The 5' end and the 3' end of the target nucleic acid molecules can be tagged with an oligonucleotide tag sequence, wherein the oligonucleotide tag sequence comprises a unique nucleotide tag. In some disclosure herein the nucleic acid molecules can be assembled de novo.

In some disclosure herein the step of determining the sequence comprises producing a sequence read using a next generation sequencing platform. In some disclosure herein the nucleic acid molecules can have a length greater than a sequence read length limit Lmax imposed by the next generation sequencing platform. In some disclosure herein each nucleic acid fragment generated can have on average a finite probability of being less than the length Lmax.

In some disclosure herein fragmentation results in the generation of a plurality of junction breaks wherein each side of junction breaks is tagged with correlated oligonucleotide barcodes sufficient to identify an upstream side and downstream side of the junction break.

In some disclosure herein the nucleic acid molecules have a length greater than 1 kbases or greater than 2 kbases.

In some disclosure herein, the method for producing nucleic acid molecules having a predetermined sequence comprises providing a pool of nucleic acid molecules comprising at least two populations of nucleic acid molecules, each population of nucleic acid molecules having a unique target nucleic acid sequence, and one or more transpososomes, wherein each transpososome has a different unique double-stranded oligonucleotide barcode. In some disclosure herein the method further comprises allowing the transpososomes to generate one or more nucleic acid junction breaks thereby generating a plurality of nucleic acid fragments comprising an oligonucleotide tag sequence at their 5' end and at their 3' end, wherein the oligonucleotide tag sequence comprises a unique nucleotide tag. The method can further comprise determining the sequence of the tagged nucleic acid fragments. In some disclosure herein each transpososome can introduce separate correlated barcodes upstream and downstream of the junction break.

In some disclosure herein the method comprises contacting a pool of nucleic acid molecules with at least one transpososome, wherein the transpososome introduces a unique double-stranded oligonucleotide sequence comprising two correlated barcodes separated by one or more cleavage sites to the nucleic acid molecules and cleaving the nucleic acid molecules. The cleavage sites can be, for example without limitation, restriction sites for restriction nucleases or meganucleases, or CRISPR sites, and the nucleic acid molecules can be cleaved with a nuclease.

In some disclosure herein the method comprises contacting a pool of nucleic acid molecules with at least one transpososome, wherein the transpososome introduces a unique double-stranded oligonucleotide sequence comprising two correlated barcodes separated by one or more dU bases to the nucleic acid molecules and cleaving the nucleic acid molecules. The nucleic acid molecules can be cleaved with a Uracil-Specific Excision Reagent.

In some disclosure herein the method comprises providing a pool of nucleic acid molecules comprising at least two populations of nucleic acid molecules, each population of nucleic acid molecules having a unique target nucleic acid sequence, the target nucleic acid sequence having a 5' end and a 3' end, and tagging the 5' end and the 3' end of the target nucleic acid molecules with an oligonucleotide tag sequence, wherein the oligonucleotide tag sequence comprises a unique nucleotide tag.

In some disclosure herein, the nucleic acid molecules are assembled de novo. In some disclosure herein, the nucleic acid molecules are synthetic nucleic acid molecules.

In some disclosure herein, the step of determining the sequence comprises producing a sequence read using a next generation sequencing platform. In some disclosure herein, the nucleic acid molecules can have a length greater than a sequence read length limit Lmax imposed by the next generation sequencing platform. In some disclosure herein, each fragment can have on average a finite probability of being less than the length Lmax.

In some disclosure herein, the method comprises generating a plurality of junction breaks wherein each side of junction breaks is tagged with correlated oligonucleotide barcodes sufficient to identify an upstream and downstream side of the junction breaks.

In some disclosure herein, the nucleic acid molecules have a length greater than 1 kbases or greater than 2 kbases.

In some disclosure herein, the pool of nucleic acid molecules can comprise error-free and error-containing nucleic acid molecules. In some disclosure herein the method comprises isolating the error-free nucleic acid molecules having the predetermined sequence.

In some disclosure herein the method comprises amplifying the nucleic acid fragments.

In some disclosure herein the method comprises amplifying error-free nucleic acid molecules having the predetermined sequence using primers having a sequence complementary to a sequence of the 5' end and the 3' end oligonucleotide tags. In some disclosure herein, the methods further comprise isolating error-free nucleic acid molecules having the predetermined sequence.

In some disclosure herein the method for producing nucleic acid molecules having a predetermined sequence comprises the steps of providing a pool of nucleic acid molecules comprising at least two populations of nucleic acid molecules, the pool of nucleic acid molecules comprising error-free and error-containing nucleic acid molecules and wherein each population of nucleic acid molecule has a unique target nucleic acid sequence having a 5' end and a 3' end; tagging the 5' end and the 3' end of the target nucleic acid molecules with an oligonucleotide tag sequence, wherein the oligonucleotide tag sequence comprises a unique nucleotide tag, thereby forming tagged target nucleic acid molecules; and diluting the tagged target nucleic acid molecules to generate a pool diluted tagged target molecules comprising an error-free tagged target nucleic acid molecules,. The method can further comprises providing one or more transpososomes, wherein each transpososome has a different unique double-stranded oligonucleotide barcode, adding the transpososomes to the pool of tagged nucleic acid molecules and allowing the transpososomes to generate one or more nucleic acid junction breaks thereby generating a plurality of nucleic acid fragments comprising an oligonucleotide tag sequence at their 5' end and at their 3' end, wherein the oligonucleotide tag sequence comprises a unique nucleotide tag. The sequence of the tagged nucleic acid fragments can then be determined, and the error-free nucleic acid molecules having the predetermined sequence can be isolated.

In some disclosure herein following the diluting step, the tagged target nucleic acid molecules can be amplified. In some embodiments, the tagged target nucleic acid molecules can be diluted and re-amplified.

Disclosure herein relates to methods for preparing nucleic acid molecules. In some disclosure herein the method comprises the step of providing one or more transpososomes and a pool of different synthetic nucleic acid molecules, each synthetic nucleic acid molecule having a unique target nucleic acid sequence, each transpososome having a different unique double-stranded oligonucleotide barcode.

The transpososomes and synthetic nucleic acid molecules can be contacted under conditions sufficient to generate one or more nucleic acid junction breaks, wherein each transpososome introduces separate correlated barcodes upstream and downstream of the junction break, thereby generating a plurality of nucleic acid fragments comprising a barcode at the 5' end, the 3' end or the 5' end and the 3' end. The sequence of the barcoded nucleic acid fragments can then be determined. In some disclosure herein the tagged nucleic acid of interest having the predetermined sequence can be isolated.

In some disclosure herein each target nucleic acid sequence has an oligonucleotide tag sequence at the 5' end, the 3' end, or the 5' end and 3' end, the oligonucleotide tag sequence comprising a unique nucleotide tag. In some disclosure herein the method generates a plurality of nucleic acid fragments comprising a barcode or an oligonucleotide tag sequence at the 5' end and the 3' end of the fragments.

In some disclosure herein, the method comprises (a) providing a pool of synthetic nucleic acid molecules comprising at least two different nucleic acid molecules, the pool of nucleic acid molecules comprising error-free and error-containing nucleic acid molecules and wherein each population of nucleic acid molecule has a unique target nucleic acid sequence having a 5' end and a 3' end, (b) tagging the 5' end and the 3' end of the target nucleic acid molecules with an oligonucleotide tag sequence, wherein the oligonucleotide tag sequence comprises a unique nucleotide tag, thereby forming tagged target nucleic acid molecules, (c) diluting the tagged target nucleic acid molecules to generate a pool of diluted tagged target molecules comprising at least one error-free tagged target nucleic acid molecule, (d) providing one or more transpososomes, wherein each transpososome has a different unique double-stranded oligonucleotide barcode, (e) adding the one or more transpososomes to the pool of tagged nucleic acid molecules, (f) allowing the one or more transpososomes to generate one or more nucleic acid junction breaks thereby generating a plurality of nucleic acid fragments comprising a barcode or an oligonucleotide tag sequence at the 5' end and at the 3' end, and (g) determining the sequence of the tagged nucleic acid fragments. In some disclosure herein the tagged nucleic acid of interest having the predetermined sequence can be isolated.

In some disclosure herein, the method for preparing nucleic acid molecules comprises providing a pool of different synthetic nucleic acid molecules, each synthetic nucleic acid molecules having a unique target nucleic acid sequence, wherein each target nucleic acid sequence has an oligonucleotide tag sequence at the 5' end and the 3' end, and wherein the oligonucleotide tag sequence comprises a unique nucleotide tag, subjecting the synthetic nucleic acid molecules to fragmentation to generate nucleic acid fragments, wherein each nucleic acid fragment comprises an oligonucleotide tag sequence at the 5' end, the 3' end, or the 5' end and the 3' end; and determining the sequence of the tagged nucleic acid fragments. In some disclosure herein the tagged nucleic acid of interest having the predetermined sequence can be isolated.

### BRIEF DESCRIPTION OF THE FIGURES

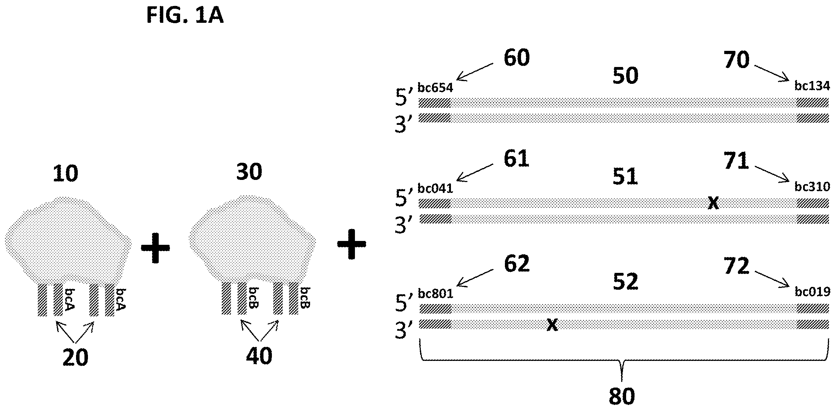

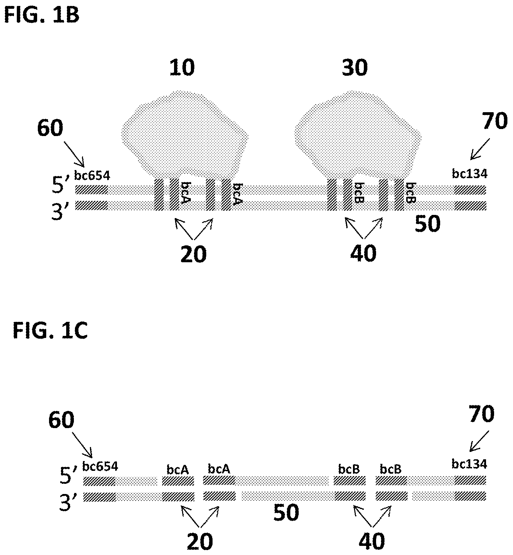

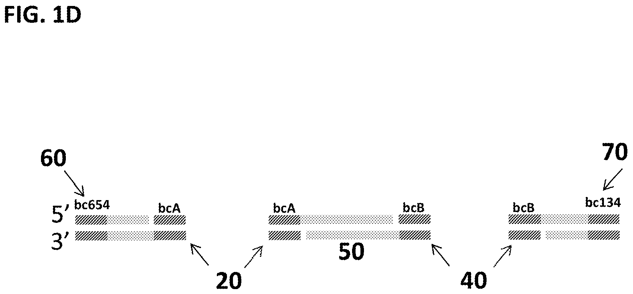

FIGs 1A-1D illustrate a schematic representation of a non-limiting exemplary method for production of long sequence verified polynucleotide constructs using a transpososome with two unconnected polynucleotide barcodes to create and tag break junctions in a long polynucleotide construct. The "x" designates incorrect or undesired sequence site. FIG. 1A illustrates the addition of transpososomes (10, 30) to tagged nucleic acids (50, 51, 52) according to some disclosure herein. FIG. 1B illustrates the mixture of tagged nucleic acids and transpososomes according to some disclosure herein. FIG. 1C illustrates the fragmentation of the nucleic acids according to some disclosure herein. FIG. 1D illustrates the tagged nucleic acid fragments according to some disclosure herein.

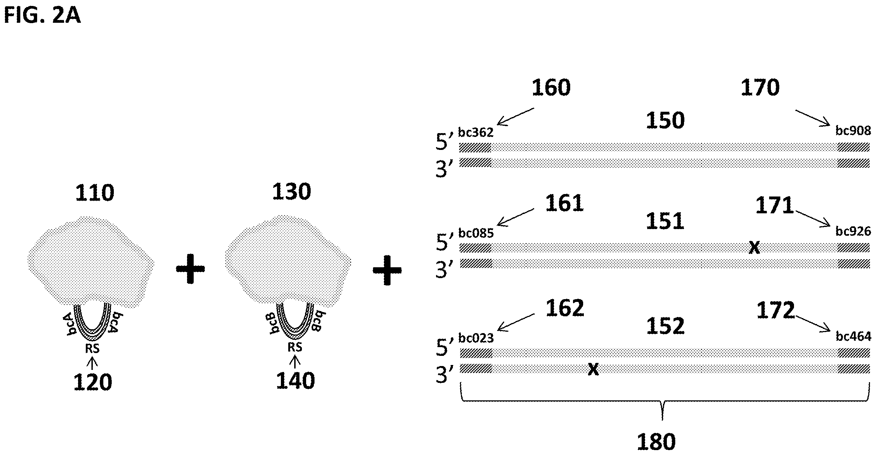

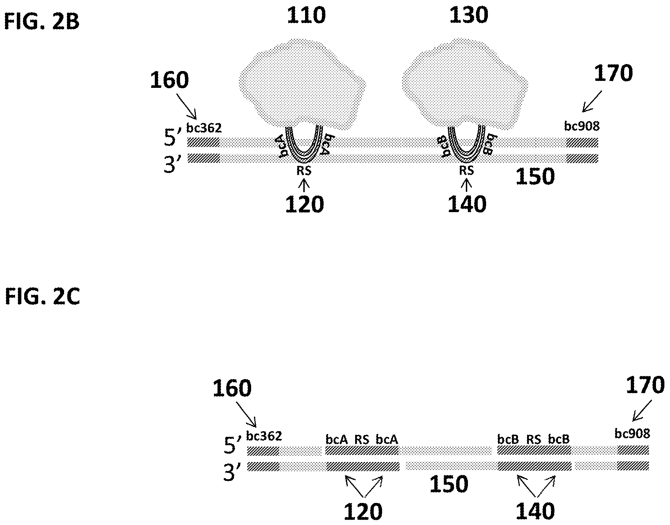

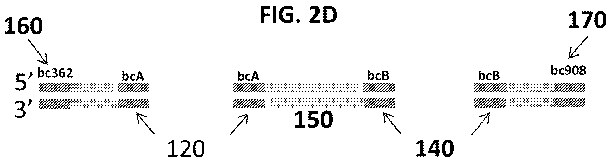

FIGS. 2A-2D illustrate a schematic representation of a non-limiting exemplary method for production of long sequence verified polynucleotide constructs using a transpososome with a single polynucleotide construct comprising two co-joined barcodes with a cleavage site (RS) in between the two barcodes. The "x" designates incorrect or undesired sequence site. FIG. 2A illustrates the addition of transpososomes (110, 130) to tagged nucleic acids (150, 151, 152) according to some disclosure herein. FIG. 2B illustrates the mixture of tagged nucleic acids and transpososomes according to some disclosure herein. FIG. 2C illustrates the fragmentation of the nucleic acids according to some disclosure herein. FIG. 2D illustrates the tagged nucleic acid fragments according to some disclosure herein.

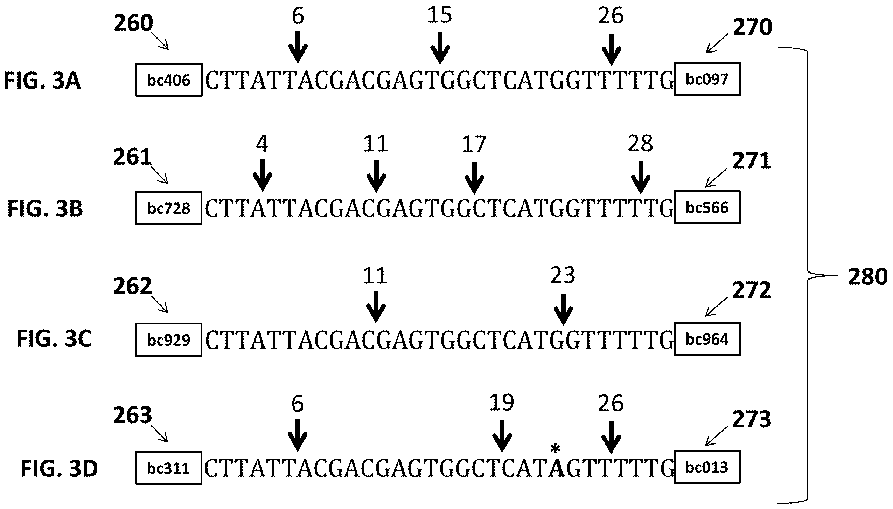

FIGS. 3A-3D illustrate a schematic representation of a non-limiting exemplary method for production of long sequence verified polynucleotide constructs using a random cutting of polynucleotides that have been labeled with 5' end (left) and 3' end (right) barcodes such that the random cuts act as unique or semi-unique identifying markers for the polynucleotide. FIG. 3A illustrates a first polynucleotide (SEQ ID NO: 1) with the position of the cut sites. FIG. 3B illustrates a second polynucleotide (SEQ ID NO: 2) with the position of the cut sites. FIG. 3C illustrates a third polynucleotide (SEQ ID NO: 3) with the position of the cut sites. FIG. 3D illustrates a fourth polynucleotide (SEQ ID NO: 4) with the position of the cut sites.

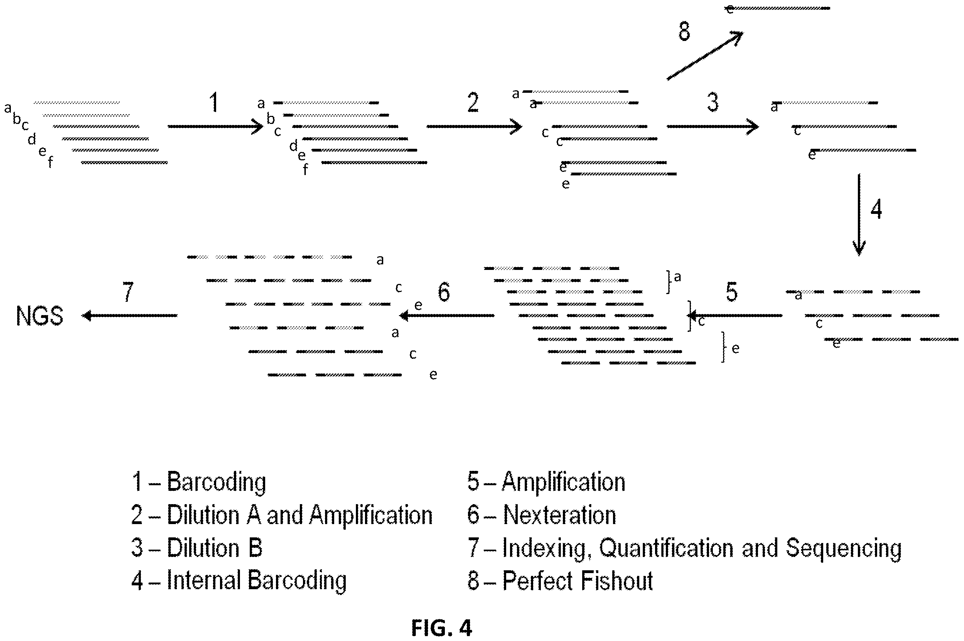

FIG. 4 illustrates a non-limiting representation of a process flow according to some disclosure herein.

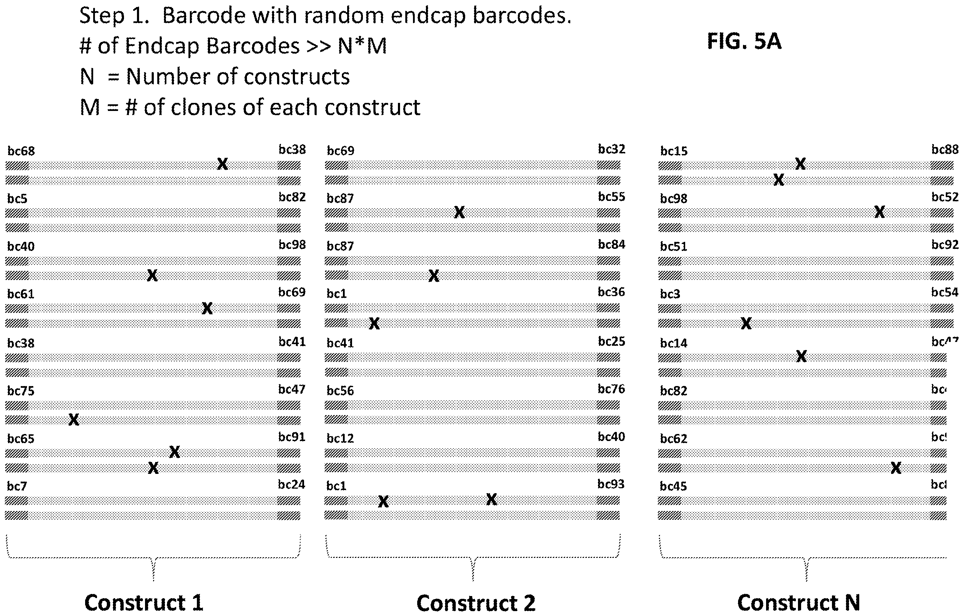

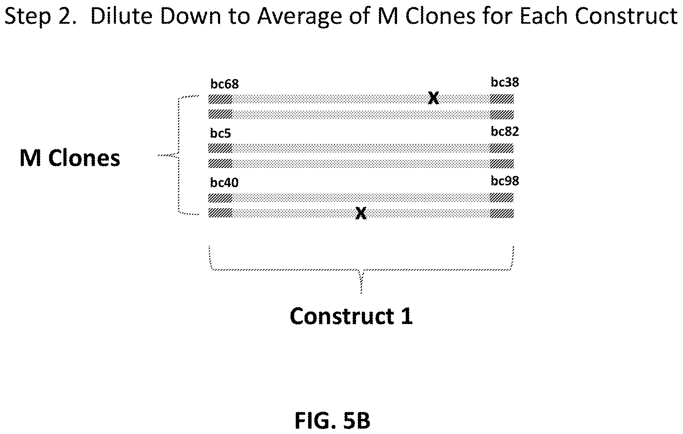

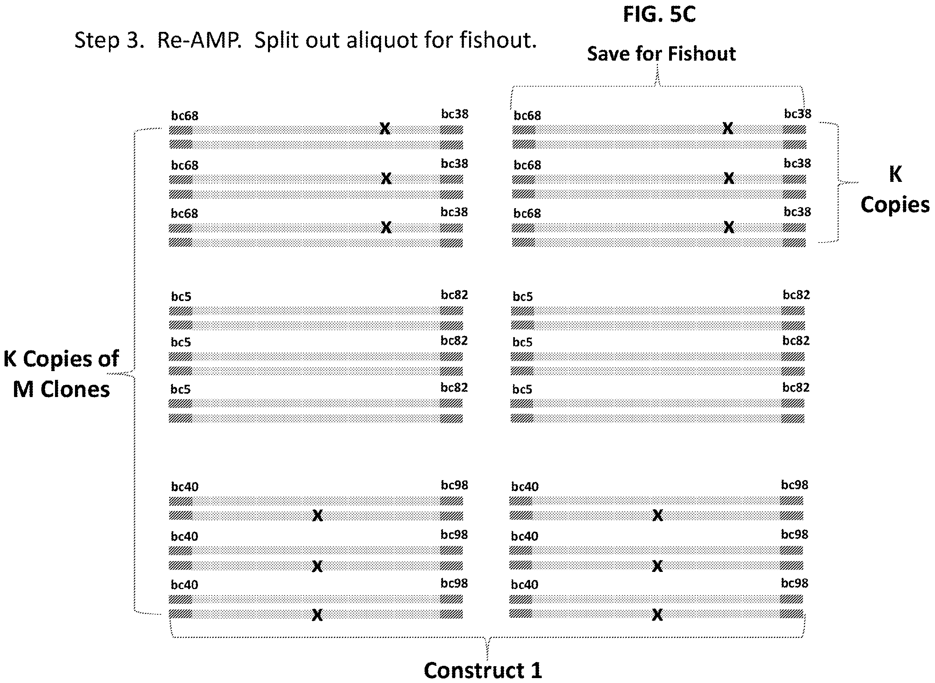

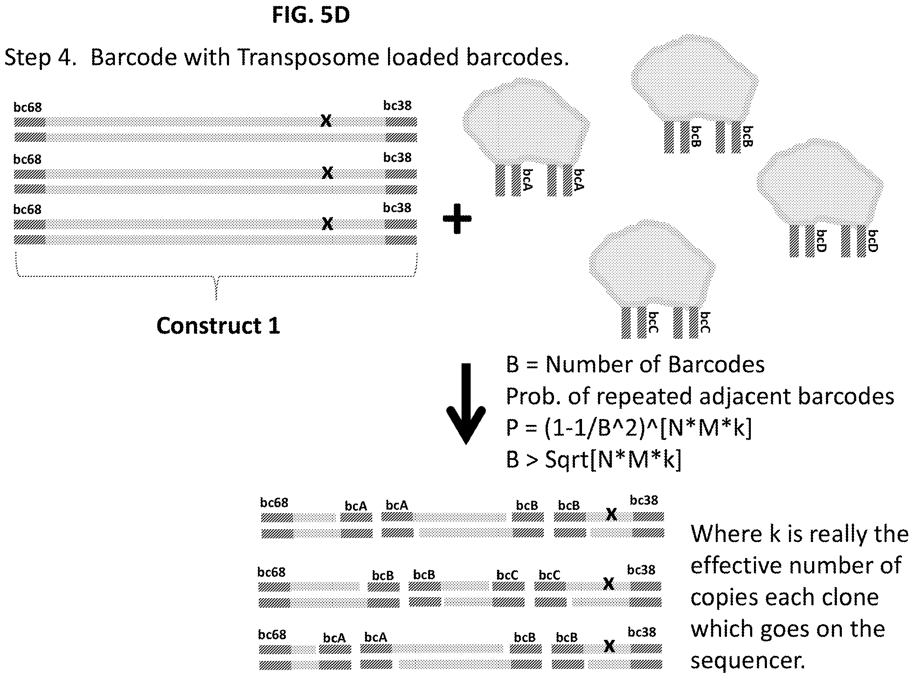

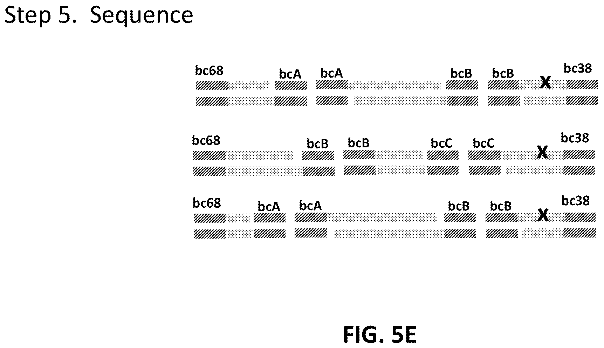

FIGS. 5A-5E is a non-limiting schematic representation of steps of the process flow. The symbol "x" in a sequence denotes a sequence error in the nucleic acid molecule. FIG. 5A illustrates the barcoding of constructs 1, 2, ..., N using random endcap barcodes , (bc). FIG. 5B illustrates the dilution step to an average of M clones. FIG. 5C illustrates the amplification step and the split out of an aliquot for fishout. FIG. 5D illustrates the barcoding of constructs using transpososomes loaded barcodes. FIG. 5E illustrates the sequencing step.

### DETAILED DESCRIPTION OF THE INVENTION

Techniques have been developed for de novo nucleic acid assembly whereby nucleic acids are made (e.g., chemically synthesized) and assembled to produce longer target nucleic acids of interest. For example, different multiplex assembly techniques are being developed for assembling oligonucleotides into larger synthetic nucleic acids. Currently there is significant interest in the synthesis of long polynucleotides in the range of more than 1Kb, 2 Kb or greater. However, one limitation of currently available assembly techniques is the relatively high error rate. Once synthesized there is a need to verify that the final nucleic acid construct has the correct sequence and in many cases to guarantee that the final construct is clonal. There is therefore a need to isolate error free nucleic acid constructs having a predetermined sequence and discarding constructs having nucleic acid errors.

Conventional methods for such verification comprise cloning the construct followed by sequencing. Recently methods have been described for producing sequence verified clonal short polynucleotide constructs (<∼ 1 kB) without the need for cloning. The methods described in U.S. Applications Serial Number 13/986,366 and 13/986,368 use unique barcodes, at the 5' and/or 3' ends of multiple candidate constructs. The pool of candidate constructs is then amplified and sequenced using next generation sequencing (NGS). Constructs which are verified to be sequence perfect can then be amplified up out of the pool based on their unique barcodes. This technique is efficient and low cost but may be limited to constructs which are of a length shorter or equal to the upper limit Lₘₐₓ of the amplification technique employed by the next generation sequencing technique being used to sequence the candidate construct pool. As an example a leading NGS platform (Illumina) is based on bridge amplification and is limited to constructs of less than Lₘₐₓ ∼ 1 Kb.

### Preparative in vitro cloning (IVC) methods

Provided herein are preparative in vitro cloning methods or strategies for de novo high fidelity nucleic acid synthesis. In some disclosure herein the in vitro cloning methods can use oligonucleotide tags. Yet in other disclosure herein the in vitro cloning methods do not necessitate the use of oligonucleotide tags.

In some disclosure herein the methods described herein allow for the cloning of nucleic acid sequences having a desired or predetermined sequence from a pool of synthetic nucleic acid molecules. In some disclosure herein the methods may include analyzing the sequence of target nucleic acids for parallel preparative cloning of a plurality of target nucleic acids. For example, the methods described herein can include a quality control step and/or quality control readout to identify the nucleic acid molecules having the correct sequence.

One skilled in the art will appreciate that the methods described herein can bypass the need for cloning via the transformation of cells with nucleic acid constructs in propagatable vectors (i.e. in vivo cloning). In addition, the methods described herein can eliminate the need to amplify candidate constructs separately before identifying the target nucleic acids having the desired sequences.

It should be appreciated that after oligonucleotide assembly, the assembly product may contain a pool of sequences containing correct and incorrect assembly products. The errors may result from sequence errors introduced during the oligonucleotide synthesis, or during the assembly of oligonucleotides into longer nucleic acids. In some instances, up to 90% of the nucleic acid sequences may contain sequence errors and be unwanted sequences. Devices and methods to selectively isolate nucleic acids having a correct predetermined sequence from nucleic acids having an incorrect sequence are provided herein. The nucleic acids having a correct sequence may be isolated by selectively isolating the nucleic acids having the correct sequence(s) from the nucleic acid having the incorrect sequences as by selectively moving or transferring the desired assembled polynucleotide of predefined sequence to a different feature of the support, or to another support (e.g. plate). Alternatively, polynucleotides having an incorrect sequence can be selectively removed from the feature comprising the polynucleotide of interest having the correct sequence.

In some disclosure herein each nucleic acid molecule can be tagged by adding a unique barcode or pair of unique barcodes to each end of the molecule. In some disclosure herein diluting the nucleic acid molecules prior to attaching the oligonucleotide tags can allow for a reduction of the complexity of the pool of nucleic acid molecules thereby enabling the use of a library of barcodes of reduced complexity. In some disclosure herein the tagged molecules can be amplified before fragmentation. Yet in other disclosure herein the tagged molecules are amplified after fragmentation. In some disclosure herein, the oligonucleotide tag sequence can comprise a primer binding site for amplification.

In some disclosure herein the oligonucleotide tag sequence can be used as a primer-binding site. Amplified tagged molecules can be subjected to fragmentation and subjected to paired-read sequencing to associate barcodes with the desired target sequence. The barcodes can be used as primers to recover the sequence clones having the desired sequence. Amplification methods are well known in the art. Examples of enzymes with polymerase activity which can be used for amplification by PCR are NA polymerase (Klenow fragment, T4 DNA polymerase), heat stable DNA polymerases from a variety of thermostable bacteria (Taq, VENT, Pfu or Tfl DNA polymerases) as well as their genetically modified derivatives (TaqGold, VENTexo, Pfu exo), or KOD Hifi DNA polymerases. In some disclosure herein amplification by chimeric PCR can be used to reduce signal to noise of barcode association.

In some disclosure herein the methods further comprise fragmenting the tagged source molecules and sequencing using MiSeq®, HiSeq® or higher throughput next generation sequencing platforms. With high throughput sequencing, enough coverage can be generated to reconstruct the consensus sequence of each tag pair construct and determine if the sequence is correct (i.e. error-free sequence).

In some disclosure herein one read of each read pair is used for sequencing barcoded end. The read pairs without any barcodes can be filtered out. Sequencing error rate can be removed by consensus calling. Nucleic acid molecules having the desired sequence can be isolated, for example, using the barcodes as primers.

As used herein, a "clonal nucleic acid" or "clonal population" or "clonal polynucleotide" are used interchangeably and refer to a clonal molecular population of nucleic acids, i.e. to nucleic acids that are substantially or completely identical to each other. In some disclosure herein the nucleic acid sequences (construction oligonucleotides, polynucleotide constructs, assembly intermediates or assembled nucleic acid of interest) may first be diluted in order to obtain a clonal population of target nucleic aids (i.e. a population containing a single target nucleic acid sequence). Accordingly, the dilution based protocol provides a population of nucleic acid molecules being substantially identical or identical to each other. In some disclosure herein the polynucleotides can be diluted serially. The concentration and the number of molecules can be assessed prior to the dilution step and a dilution ratio can be calculated in order to produce a clonal population.

In some disclosure herein the tagged molecules are diluted down to an average number of clones for each construct. The diluted tagged molecules can be amplified. In some disclosure herein the amplified tagged molecules are diluted prior to be subjected to internal barcoding, as described herein.

### Production of long sequence verified polynucleotide constructs

Disclosure herein can be used for the clone free production of clonal sequence verified long length (> 1 Kb) polynucleotides.

In some disclosure herein, methods for the clone free production of clonal sequence verified long length (> 1 Kb) polynucleotides are provided. In some disclosure herein polynucleotide constructs of length greater than Lmax can be assembled. In some disclosure herein the methods comprise labeling the ends of each assembled polynucleotide or construct with unique barcodes. In some disclosure herein the barcoded constructs can be fragmented into fragments having a size inferior to Lmax in a manner in which each side of the break junctions is labeled with additional correlated polynucleotide barcodes. One of skill in the art will appreciate that using such approach, it is possible to determine the sequence of the original long sequence. In some disclosure herein, the polynucleotides which are determined to have the correct sequence may be amplified out of a pool by means of their unique 5' and 3' barcodes.

Yet in some disclosure herein, after assembling polynucleotide constructs of length greater than Lmax, the ends of each assembled polynucleotide can be labeled with unique barcodes. In some disclosure herein, the barcoded long construct can be fragmented into fragments having a size inferior to Lmax in which the internal break points are at random locations. The particular random point of breakage can act as a unique (or semi-unique) label for a particular molecule such that the fragments can be sequenced starting from either the left or right barcode. Using such approach it is possible to determine the sequence of the original long sequence. In a subsequent step, the polynucleotides which are determined to have the correct sequence may be amplified out of a pool by means of their unique 5' end (left) and 3' end (right) barcodes.

Disclosure herein can be used to isolate nucleic acid molecules from large numbers of nucleic acid fragments efficiently, and/or to reduce the number of steps required to generate large nucleic acid products, while reducing error rate. Disclosure herein can be incorporated into nucleic assembly procedures to increase assembly fidelity, throughput and/or efficiency, decrease cost, and/or reduce assembly time. Disclosure herein may be automated and/or implemented in a high throughput assembly context to facilitate parallel production of many different target nucleic acid products. In some disclosure herein nucleic acid constructs may be assembled using starting nucleic acids obtained from one or more different sources (e.g., synthetic or natural polynucleotides, nucleic acid amplification products, nucleic acid degradation products, oligonucleotides, etc.). Disclosure herein relates to the use of a high throughput platform for sequencing nucleic acids such as assembled nucleic acid constructs to identify high fidelity nucleic acids at lower cost. Such platform has the advantage to be scalable, to allow multiplexed processing, to allow for the generation of a large number of sequence reads, to have a fast turnaround time and to be cost efficient.

The methods described herein may be used with any nucleic acid molecules, library of nucleic acids or pool of nucleic acids. For example, the methods of the disclosure can be used to generate nucleic acid constructs, oligonucleotides or libraries of nucleic acids having a predefined sequence. In some disclosure herein the nucleic acid library may be obtained from a commercial source or may be designed and/or synthesized onto a solid support (e.g. array).

In some disclosure herein each nucleic acid fragment or construct (also referred herein as nucleic acid of interest) being assembled may be between about 100 nucleotides long and about 5,000 nucleotides long (e.g., about 200, about 300, about 400, about 500, about 600, about 700, about 800, about 900, about 1,000, about 2,000, about 3,000, about 4,000 or 5,000 or more nucleotides long). However, longer (e.g., about 5,500 or more nucleotides long, about 7,500 or more nucleotides long, about 10,000 or more nucleotides long, etc.) or shorter nucleic acid fragments may be assembled using an assembly technique (e.g., shotgun assembly into a plasmid vector). It should be appreciated that the size of each nucleic acid fragment may be independent of the size of other nucleic acid fragments added to an assembly. However, in some disclosure herein each nucleic acid fragment may be approximately the same size.

Disclosure herein relates to methods and compositions for the selective isolation of nucleic acid constructs having a predetermined sequence of interest. As used herein, the term "predetermined sequence" or "predefined sequence" means that the sequence of the polymer is known and chosen before synthesis or assembly of the polymer. In particular, disclosure herein is described herein primarily with regard to the preparation of nucleic acids molecules, the sequence of the oligonucleotide or polynucleotide being known and chosen before the synthesis or assembly of the nucleic acid molecules. In some disclosure herein of the technology provided herein, immobilized oligonucleotides or polynucleotides are used as a source of material. In various disclosure herein the methods described herein use pluralities of construction oligonucleotides, each oligonucleotide having a target sequence being determined based on the sequence of the final nucleic acid constructs to be synthesized (also referred herein as nucleic acid of interest or target nucleic acid). In disclosure herein, oligonucleotides are short nucleic acid molecules. For example, oligonucleotides may be from 10 to about 300 nucleotides, from 20 to about 400 nucleotides, from 30 to about 500 nucleotides, from 40 to about 600 nucleotides, or more than about 600 nucleotides long. However, shorter or longer oligonucleotides may be used. Oligonucleotides may be designed to have different length. In some disclosure herein the sequence of the polynucleotide construct may be divided up into a plurality of shorter sequences (e.g. construction oligonucleotides) that can be synthesized in parallel and assembled into a single or a plurality of desired polynucleotide constructs using the methods described herein. Nucleic acids, such as construction oligonucleotides, may be pooled from one or more arrays to form a library or pool of nucleic acids before being processed (e.g. tagged, diluted, amplified, sequenced, isolated, assembled etc.).

According to disclosure herein, each nucleic acid sequence to be assembled (also referred herein as nucleic acid source molecules) can comprise an internal predetermined target sequence having a 5' end and a 3' end and additional flanking sequences at the 5' end and/or at the 3' end of the internal target sequence. In some disclosure herein the internal target sequences or nucleic acids including the internal target sequences and the additional 5' and 3' flanking sequences can be synthesized onto a solid support as described herein.

In some disclosure herein the synthetic nucleic acid sequences comprise an internal target sequence and non-target sequences upstream and downstream the target sequence. In some disclosure herein the nucleic acid sequences can be synthesized with additional sequences, such as oligonucleotide tag sequences. For example, the nucleic acid sequences can be designed so that they include an oligonucleotide tag sequence chosen from a library of oligonucleotide tag sequences, as described herein. In some disclosure herein the nucleic acid sequences can be designed to have an oligonucleotide tag sequence including a sequence common across a set of nucleic acid constructs. The term "common sequence" means that the sequences are identical. In some disclosure herein the common sequences can be universal sequences. Yet in other disclosure herein the 5' oligonucleotide tag sequences are designed to have common sequences at their 3' end and the 3' oligonucleotide tag sequences are designed to have common sequences at their 5' end. For example, the nucleic acid can be designed to have a common sequence at the 3' end of the 5' oligonucleotide tag and at the 5' end of the 3' oligonucleotide tag. The library of oligonucleotide tag sequences can be used for nucleic acid construct to be assembled from a single array. Yet in other disclosure herein the library of oligonucleotide tags can be reused for different constructs produced from different arrays. In some disclosure herein the library of oligonucleotide tag sequences can be designed to be universal. In some disclosure herein, the nucleic acid or the oligonucleotide tags are designed to have additional sequences. The additional sequences can comprise any nucleotide sequence suitable for nucleic acid sequencing, amplification, isolation or assembly in a pool.

Disclosure herein relates to compositions for sorting and/or cloning nucleic acids having a desired predetermined sequence. In some disclosure herein composition comprising a synthetic nucleic acid molecule having a 5' portion comprising a tag and a 3' portion comprising a tag are provided. In some disclosure herein the composition comprises a plurality of synthetic nucleic acid molecules, wherein the nucleic acid molecules comprise 5' portions comprising a tag that differ by at least one nucleotide, and 3' portions comprising a tag that differ by at least one nucleotide. In some disclosure herein the tag comprises one or more of a restriction site domain, a capture tag, a sequencing tag, an amplification tag, a detection tag, cleavage site, and barcode.

In some disclosure herein, the composition comprises a plurality of synthetic nucleic acids and a plurality of transpososomes. In some disclosure herein the composition comprises a transposase. In some disclosure herein the transpososomes can be loaded with one or more barcodes. In some disclosure herein, each transpososome can be loaded with a unique barcode having a unique predetermined sequence. For example, each transpososome can be loaded with two identical nucleic acid barcodes. In some disclosure herein the barcodes are not connected to each other. Yet in other disclosure herein, the barcodes can be connected with each other.

In some disclosure herein, the transposase is selected from a Tn5 transposase, a Ty transposase, a Sleeping Beauty transposase, a piggyback transposase, and a Mu transposase. In some disclosure herein, the nucleic acids and the transpososomes are provided in a mixture.

In some disclosure herein, kits comprising any of the compositions described above or elsewhere herein are provided. In some disclosure herein, the kit further comprises reagents for an amplification reaction. In some disclosure herein, a kit further comprises reagents for a DNA sequencing reaction.

### Production of long sequence verified polynucleotide constructs using transpososomes

In some disclosure herein, the methods use a functional nucleic acid-protein complex capable of transposition.

Referring to FIG. 1A-1D, non-limiting methods are shown for the production of long sequence verified polynucleotide constructs using transpososomes with two unconnected polynucleotide barcodes to create and tag break junctions in a long polynucleotide construct which may then be used to recreate the full sequence of the long polynucleotide. The "x" designates incorrect or undesired sequence site.

In some disclosure herein the transpososome can be comprised of at least a transposase enzyme and a transposase recognition site. In some disclosure herein the transpososome is associated at least one barcode. In some disclosure herein the barcodes can be identical on each transpososome. According to some disclosure herein, the method can comprise using a plurality of transpososomes, each transpososome having one or more identical barcodes, such that the plurality of transpososomes have a plurality of barcodes. According to the methods described herein, the plurality of transpososomes can be mixed with one or more nucleic acids in a pool. Under appropriate conditions, the transpososomes can fragment the nucleic acids leading to the incorporation of the barcodes and the generation barcoded nucleic acid fragments. In some disclosure herein the barcoded nucleic acid fragments can be amplified.

In some disclosure herein the barcodes are double-stranded oligonucleotide tags and each oligonucleotide tag has a unique sequence. In some disclosure herein upon fragmentation with the barcoded transpososome, both sides (upstream and downstream) of the junction break or cut are labeled with correlated barcodes. In some disclosure herein the 3' end of a first fragment sequence is labeled with the same barcode that the 5' end of the next downstream fragment. Accordingly, the original sequence of the long polynucleotide may be reconstructed.

In some disclosure herein the method further comprises the step of contacting the barcoded fragments with DNA polymerase so that fully double-stranded nucleic acid molecules are produced from the fragments. This step can be used to fill the gaps generated in the transposition products in the transposition reaction.

Referring to FIG. 1A, transpososomes (10, 30) can be labeled with pairs of identical or associated double-stranded oligonucleotide barcodes (20, 40) respectively. Labeled transpososomes (bcA, bcB)can be added to a pool or ensemble (80) of long polynucleotide constructs (50, 51, 52) with 5' (left, 60, 61, 62) and 3' (right, 70, 71, 72) barcodes that uniquely label and identify each long polynucleotide construct (50, 51, 52) from other long polynucleotide constructs in the pool (80).

Referring to FIG. 1B and FIG. 1C, transpososomes randomly contact the long polynucleotide constructs (1b) and then cut creating barcode label (20, 40) members (50) of the long polynucleotide ensemble. Referring to FIG. 1D, this fragmentation produces fragments having a size that can be sequenced using Next Generation Sequencing (e.g. less than 500 bps) which may be sequenced using next generation sequencing platform (e.g. Illumina platform). Since the action of the transpososome is to label both sides of a cut site with correlated barcodes (in this example, bcA and bcB) the original sequence of the long polynucleotide may be reconstructed. Referring to FIG. 1D, a first fragment has the 5'end left barcode bc654 which goes with the 3'end right barcode bcA, a second fragment has the 5'end left barcode bcA which goes with the 3'end right barcode bcB and the third fragment has the 5'end left barcode bcB which goes with the 3'end right barcode bc134 allowing the original full length polynucleotide construct to be reconstructed. Polynucleotides which are determined to have the correct sequence may be amplified out of the pool (80) by means of their unique left (60) and right (70) barcodes.

It should be appreciated that in the methods described above, there should be a sufficient number of transpososomes with different correlated barcode pairs such that the probability of any long polynucleotide in the ensemble (80) sharing more than 1 barcode with any other long polynucleotide in the ensemble is small. In an exemplary disclosure herein assuming the ensemble (80) contains 100 different polynucleotide sequences with a goal of 10 clones for each one and assuming an average of 2 cuts per long polynucleotide then there should be > 32 (= Sqrt[100*10]) different correlated barcode pair transpososomes (10, 20) for use with that long polynucleotide ensemble (80).

Referring to FIG. 2, as the number of members of the ensemble of long polynucleotide constructs (180) becomes large, the number of required different correlated barcode pair transpososomes becomes large (scaling as the square root of the number of members of the ensemble of long polynucleotide constructs). As an example, assuming the ensemble (180) contains 1,000 different polynucleotides with 10 clones for each one and assuming an average of 2 cuts per long polynucleotide, there should be > 100 (= Sqrt[1000*10]) different correlated barcode pair transpososomes for use with the long polynucleotide ensemble (180).

In some disclosure herein, in order to simplify the preparation of large number of different correlated barcode pair transpososomes, such transpososomes (110, 120) may be prepared by linking two co-joined barcodes (e.g. bcA and bcA, FIG. 2A) with a cleavage site (RS) in between the two barcodes (120, 140) as opposed to separate correlated barcodes as described in FIG. 1. In some disclosure herein the entire set of required co-joined barcodes (120,140) may be attached to their respective transpososome (110, 130) in a single reaction.

Referring to FIG. 2(a), transpososomes (110,130) can be labeled with pairs of identical or associated double-stranded oligonucleotide barcodes (120,140) respectively. In some disclosure herein the transpososomes can be added to an ensemble (180) of long polynucleotide constructs (150, 151, 152) with 5' end (left, 160, 161, 162) and 3' end (right, 170, 171, 172) barcodes which uniquely label and identify each long polynucleotide constructs (150, 151, 152) from other long polynucleotide constructs in the ensemble (180).

Referring to FIG. 2B, transpososomes can randomly contact members (150) of the long polynucleotide ensemble. The transpososomes can then cut and barcode label (120, 140) members (150) of the long polynucleotide ensemble (FIG. 2C). Referring to FIG. 2D, cleavage at the cleavage sites (e.g. after contacting with a restriction enzyme or other means of cleaving the restriction sites (RS)) produces fragments having a size which may be sequenced using next generation sequencing (e.g. less than 500 bp for Illumina platform). In some disclosure herein the cleavage site may be the incorporation of a uracil base or a restriction site. In some disclosure herein mixture of Uracil DNA glycosylase (UDG) and the DNA glycosylase-lyase Endonuclease VIII, such as USER™ (Uracil-Specific Excision Reagent) can be used to cleave sequences comprising one or more dU bases.

Since the action of the transpososome is to label both sides of a cut site with correlated barcodes (referring to FIG. 2A, with bcA and bcB) the original sequence of the long polynucleotide may be reconstructed. Referring to FIG. 2D, action of the transpososome generates a first fragment having the 5'end left barcode bc362 which goes with the 3'end right barcode bcA, a second fragment having the 5'end left barcode bcA which goes with the 3'end right barcode bcB and a third fragment having the 5'end left barcode bcB which goes with the right barcode bc908, allowing the original full length polynucleotide construct to be reconstructed. Polynucleotides which are determined to have the correct sequence may be amplified out of the ensemble (180) by means of their unique left (160) and right (170) barcodes.

It should be appreciated that in the above process, there should be a sufficient number of transpososomes with different correlated barcode pairs such that the probability of any long polynucleotide in the ensemble (180) sharing more than 1 barcode with any other long polynucleotide in the ensemble is small. In an exemplary disclosure herein assuming the ensemble (180) contains 1000 different polynucleotides with 10 clones for each one and an average of 2 cuts per long polynucleotide, there should be > 100 (= Sqrt[1000*10]) different correlated barcode pair transpososomes (110, 120) for use with that long polynucleotide ensemble (180).

Referring to FIG. 3, methods for the production of long sequence verified polynucleotide constructs that do not require explicit correlated barcode labeling of internal cut sites are described. Referring to FIG. 3, an ensemble of long polynucleotides (280) have attached to them unique left (260, 261, 262, 263) and right (270, 271, 272, 273) barcodes. Such long polynucleotides can then randomly cut (e.g. by a transposase) such that the average size of resulting fragments constitute a size appropriate for next generation sequencing (e.g. 500 bp for Illumina bridge amplification base next generation sequencing). Since the desired long polynucleotide sequence is known, the location of each random cut site along the ensemble of long polynucleotides constitutes a unique or semi-unique identifier for each individual molecule. Referring to FIG. 3B, the polynucleotide has cut sites at positions 4, 11, 17 and 28. When the fragments are sequenced starting from the 5'end left barcode (261), a collection of fragments are generated (L= Left End, R = Right End):
(L:bc728, R: CTTA)
(L:TTAC,R: CGAC)
(L:GAGT, R: GTGG)
(L:CTCA, R: TTTT)
(L:TG, R: bc566).

Since it is known that in the desired sequence CTTA is followed by TTAC it can be conclude that fragment 1 in the list above is connected to fragment 2 in the list above and thus the original long polynucleotide sequence can be reconstructed. Polynucleotides which are determined to have the correct sequence may be amplified out of the ensemble (280) by means of their unique left (260) and right (270) barcodes.

However, if two long polynucleotide sequences share two or more of the same cut sites then it is not possible to tell which long polynucleotide a particular fragment came from. As an example, both the polynucleotide of FIG. 3A and the polynucleotide of FIG 3D have cut sites at base positions 6 and 26. To further illustrate the example the polynucleotide of FIG 3D has mutation error denoted by an asterisk. When the ensemble of fragments is sequenced, a collection of fragments coming from both constructs are generated: (L= Left End, R = Right End):
(L:bc406, R: TATT)
(L:bc311, R: TATT)
(L:ACGA,R: GAGT)
(L:ACGA,R: GGCT)
(L:GGCT, R:GGTT)
(L:CAT**A***,R:**A***GTT)
(L:TTTG,R:bc097)
(L:TTTG,R:bc273)

From the sequence of the fragments above it may not be possible to tell whether the mutation (**A***) comes from the polynucleotide of FIG. 3A and the polynucleotide of FIG. 3D and thus both molecules will need to be discarded as candidates to be amplified out of the pool as a perfect construct. The desire to avoid having several long polynucleotide molecules in the ensemble (280) that shares more than one cut site sets the limit for the length and diversity of an ensemble of polynucleotides that may be sequenced using this approach. Table 1 and Table 2 summarize the findings.

Table 1 assumes a maximum bridge amp length of 500 bp and a construct length of 10,000 bp. Thus an average 19 cuts is required to cut down to the size for sequencing. Assuming 10 clones per construct then there is probability of 1:84 of having two molecules with two or more of the same cut sites which is sufficient to find a large number of clones which do not share more than 1 exact cut site with any other clone in the ensemble.

**Table 1**

| | |
|---|---|
| Construct Length (bps) | 10000 |
| Max Bridge Amp Length | 500 |
| Number of Cuts | 19 |
| p = 1: | 526 |
| P1 = 1: | 28 |
| P2 = 1: | 30 |
| P1*P2 = 1: | 837 |
| | |
| Number of Clones or Similar Sequences | 10 |
| | |
| P 1: | 84 |

Table 2 assumes a maximum bridge amp length of 500 bp and a construct length of 10,000 bp. Thus an average 19 cuts is required to cut down to the size for sequencing. Assuming 100 clones per construct then there is a probability of 1:9 of having two molecules with two or more of the same cut sites which is sufficient to find a large number of clones which do not share more than 1 exact cut site with any other clone in the ensemble.

**Table 2**

| | |
|---|---|
| Construct Length | 10000 |
| Max Bridge Amp Length | 500 |
| Number of Cuts | 19 |
| p = 1: | 526 |
| P1 = 1: | 28 |
| P2 = 1: | 30 |
| P1*P2 = 1: | 837 |
| | |
| Number of Clones or Similar Sequences | 100 |
| | |
| P 1: | 9 |

Table 3 assumes a maximum bridge amp length of 1000 bp and a construct length of 100,000 bp. Thus an average 99 cuts is required to cut down to the size for sequencing. Assuming 10 clones per construct then there is a probability of 1:12 of having two molecules with two or more of the same cut sites which is sufficient to find a large number of clones which do not share more than 1 exact cut site with any other clone in the ensemble.

**Table 3**

| | |
|---|---|
| Construct Length | 100000 |
| Max Bridge Amp Length | 1000 |
| Number of Cuts | 99 |
| p = 1: | 1,010 |
| P1 = 1: | 11 |
| P2 = 1: | 11 |
| P1*P2 = 1: | 116 |
| | |
| Number of Clones or Similar Sequences | 10 |
| | |
| P 1: | 12 |

Referring to FIG. 4 and FIG. 5, non-limiting process flow and methods are shown for the production of sequence verified long nucleic acid products using transposase-based barcoding. FIG. 4 illustrates a process flow according to some disclosure herein to produce barcoded nucleic acid fragments. According to some disclosure herein barcodes that define a fragmentation junction can be used to piece fragments back together, allowing the sequencing of longer constructs. Referring to FIG. 4, a pool of nucleic molecules can be tagged or barcoded (step 1), the tagged nucleic acid molecules can be diluted and amplified (step 2), the amplified pool of tagged molecules can be diluted (step 3), the diluted tagged molecules can be fragmented and tagged with barcodes to form barcoded fragments (e.g. using transpososomes/transposase, step 4), the barcoded fragments can be amplified (step 5), and digested using Nextera™ tagmentation (step 6) and sequenced using MiSeq®, HiSeq® or higher throughput next generation sequencing platforms. The Nextera™ tagmented paired reads generally generate one sequence with an oligonucleotide tag sequence for identification, and another sequence internal to the construct target region (as illustrated in FIG. 2C). With high throughput sequencing, enough coverage can be generated to reconstruct the consensus sequence of each tag pair construct and determine if the sequence is correct (i.e. error-free sequence). The polynucleotides having the error-free predetermined sequences can be sorted or fish-out according to the identity of the barcodes (step 8).

In some disclosure herein the methods comprise the following steps:
(a) providing a pool of different nucleic acid constructs (also referred herein as source molecules); (b) providing a repertoire of oligonucleotide tags, each oligonucleotide tag comprising a unique nucleotide tag sequence or barcode; (c) attaching at the 5' end and at the 3' end an oligonucleotide tag to each source molecule in the pool of nucleic acid molecules, such that substantially all different molecules in the pool have a different oligonucleotide tag pair attached thereto and so as to associate a barcode to a specific source molecule.

In some disclosure herein the barcode sequence may also act as a primer binding site to amplify the barcoded nucleic acid molecules or to isolate the nucleic acid molecules having the desired predetermined sequence. In such disclosure herein the term barcode and oligonucleotide tag can be used interchangeably. In such disclosure herein, the terms "barcoded nucleic acids" and "tagged nucleic acids" can be used interchangeably. It should be appreciated that the oligonucleotide tags may be of any suitable length and composition. In some disclosure herein the oligonucleotide tags can be designed such as (a) to allow generation of a sufficient large repertoire of barcodes to allow each nucleic acid molecule to be tagged with a unique barcode at each end; and (b) to minimize cross hybridization between different barcodes. In some disclosure herein the nucleotide sequence of each barcode is sufficiently different from any other barcode of the repertoire so that no member of the barcode repertoire can form a dimer under the reactions conditions, such as the hybridization conditions, used.

It should be appreciated that if there is a number N of constructs and there is M clones for each construct, the number of endcap barcodes used to tag the constructs should be substantially higher than N*M (FIG. 5A).

In some disclosure herein the tagged nucleic acid sequences (also referred herein as tagged constructs) can be diluted. In some disclosure herein the tagged constructs can be diluted such that the pool of constructs comprises an average of M clones for each construct (FIG. 5B). In some disclosure herein the M clones can comprise error-containing and error-free constructs.

In some disclosure herein following dilution, the tagged constructs can be amplified (FIG. 5C). In some disclosure herein the oligonucleotide tag sequence can comprise a primer binding site for amplification. In some disclosure herein, the oligonucleotide tag sequence can be used as a primer-binding site. Referring to FIG. 5C, in some disclosure herein amplification can result in K copies of the M clones and an aliquot of the amplification product can be saved for fishout or for sorting the construct having the correct desired sequence according to the identity of the barcodes (e.g. error-free target molecule).

In some disclosure herein, following amplification, the tagged constructs can be internally barcoded. In some disclosure herein, the tagged constructs can be fragmented using transpososomes loaded with barcodes. Referring to FIG. 5D, the K copies of the M clones can be mixed with transpososomes having B different barcodes, where B is superior to the square root of N*M*k, where k is the effective number of copies of each clone which is subjected to the next generation sequencing.

In some disclosure herein, a paired end read for each nucleic acid molecule can be obtained and the nucleic acid molecules having the desired predetermined sequence according to the identity of the barcodes can be sorted. In some disclosure herein, and referring to FIG. 5E, fragments size are generally kept under 800b bps and sequenced directly. Since the transpososome cuts are random locations, the sequences generated from the about 250 bps paired end read can be used for the reconstruction of the initial consensus sequence as described herein.

### Tag oligonucleotides

In some disclosure herein, the 5' end and the 3' end of each nucleic acid molecules within the pool can be tagged with a pair of tag oligonucleotide sequence. In some disclosure herein, the tag oligonucleotide sequence can be composed of common DNA primer regions and unique "barcode" regions such as a specific nucleotide sequence. In some disclosure herein, the number of tag nucleotide sequences can be greater than the number of molecules per construct (i.e. 10-1000 molecules in the dilution).

As used herein, the term "barcode" refers to a unique oligonucleotide tag sequence that allows a corresponding nucleic acid sequence to be identified. By designing the repertoire or library of barcodes to form a library of barcodes large enough relative to the number of nucleic acid molecules, each different nucleic acid molecule can have a unique barcode pair. In some disclosure herein the library of barcodes comprises a plurality of 5' end barcodes and a plurality of 3' end barcodes. Each 5' end barcode of the library can be design to have 3' end or internal sequence common to each member of the library. Each 3' end barcode of the library can be design to have 5' end or internal sequence common to each member of the library

As described herein, the nucleic acid molecules can be designed to include a disclosure herein barcode at the 5' and at the 3' ends. In some disclosure herein the barcodes can have common sequences within and across a set of constructs. For example, the barcodes can be universal for each construct assembled from a single array. In some disclosure herein the barcodes can have common junction sequences or common primer binding site sequences.

In some disclosure herein the barcode sequence may also act as a primer binding site to amplify the barcoded nucleic acid molecules or to isolate the nucleic acid molecules having the desired predetermined sequence. In such disclosure herein the term barcode and oligonucleotide tag can be used interchangeably. In such disclosure herein the terms "barcoded nucleic acids" and "tagged nucleic acids" can be used interchangeably. It should be appreciated that the oligonucleotide tags may be of any suitable length and composition. In some disclosure herein the oligonucleotide tags can be designed such as (a) to allow generation of a sufficient large repertoire of barcodes to allow each nucleic acid molecule to be tagged with a unique barcode at each end; and (b) to minimize cross hybridization between different barcodes. In some disclosure herein the nucleotide sequence of each barcode is sufficiently different from any other barcode of the repertoire so that no member of the barcode repertoire can form a dimer under the reactions conditions, such as the hybridization conditions, used.

In some disclosure herein the barcode sequence can be 6 bp, 7 bp, 8 bp, 9 bp, 10 bp, 12 bp, 13 bp, 14 bp, 15 bp, 16 bp, 17 bp, 18 bp, 19 bp, 20 bp, 21 bp, 22 bp, 23 bp, 24 bp, 25 bp, 26 bp, 27 bp, 28 bp, 29 bp, 30 bp or more than 30 bp in length. In some disclosure herein the 5' end barcode sequence and the 3' end barcode sequence can differ in length. For example, the 5' barcode can be 14 nucleotides in length and the 3' barcode can be 20 nucleotides in length. In some disclosure herein the length of the barcode can be chosen to minimize reduction in barcode space, maximize barcode space at the 3' end for primability, allows error correction for barcodes, and/or minimize the variation of barcode melting temperatures. For example, the melting temperatures of the barcodes within a set can be within 10°C of one another, within 5°C of one another or within 2°C of one another.

Each barcode sequence can include a completely degenerate sequence, a partially degenerate sequence or a non-degenerate sequence.

For example, a 6 bp, 7 bp, 8 bp, or longer nucleotide tag can be used. In some disclosure herein a degenerate sequence NNNNNNNN (8 degenerate bases, wherein each N can be any natural or non-natural nucleotide) can be used and generates 65,536 unique barcodes. In some disclosure herein the length of the nucleotide tag can be chosen such as to limit the number of pairs of tags that share a common tag sequence for each nucleic acid construct.

One of skill in the art would appreciate that a completely degenerate sequence can give rise to a high number of different barcodes but also to higher variations in primer melting temperature Tm. Melting temperature Tm is the temperature at which a population of double-stranded nucleic acid molecules becomes half dissociated into single-strands. Equations for calculating the Tm of nucleic acids are well known in the art. For example, a simple estimate of the Tm value can be calculated by the equation Tm= 81.5 ± 0.41 (%G+C) when the nucleic acid are in aqueous solution at 1M NaCl. In some disclosure herein the barcode sequences are coded barcode and may comprise a partially degenerate sequence combined with fixed or constant nucleotides.

In some disclosure herein barcode sequences can be designed, analyzed and ranked to generate a ranked list of nucleotide tags that are enriched for both perfect sequence and primer performance. It should be appreciated that the coded barcodes provide a method for generating primers with tighter Tm range.

In some disclosure herein the tag oligonucleotide sequences or barcodes can be joined to each nucleic acid molecule to form a nucleic acid molecule comprising a tag oligonucleotide sequence at its 5' end and at its 3' end. In some disclosure herein the tag oligonucleotide sequences or barcodes can be ligated to blunt end nucleic acid molecules using a ligase. For example, the ligase can be a T7 ligase or any other ligase capable of ligating the tag oligonucleotide sequences to the nucleic acid molecules. Ligation can be performed under conditions suitable to avoid concatamerization of the nucleic acid constructs. In other disclosure herein the nucleic acid molecules are designed to have at their 5' and 3' ends a sequence that is common or complementary to the tag oligonucleotide sequences. In some disclosure herein the tag oligonucleotide sequences and the nucleic acid molecules having common sequences can be joined as adaptamers by polymerase chain reaction.

### Sequencing

In some disclosure herein the target nucleic acid sequence or a copy of the target nucleic acid sequence can be isolated from a pool of nucleic acid sequences, some of them containing one or more sequence errors. As used herein, a copy of the target nucleic acid sequence refers to a copy using template dependent process such as PCR. In some disclosure herein sequence determination of the target nucleic acid sequences can be performed using sequencing of individual molecules, such as single molecule sequencing, or sequencing of an amplified population of target nucleic acid sequences, such as polony sequencing. In some disclosure herein the pool of nucleic acid molecules are subjected to high throughput paired end sequencing reactions, such as using the HiSeq®, MiSeq® (Illumina) or the like or any suitable next-generation sequencing system (NGS).

In some disclosure herein the nucleic acid molecules are amplified using the common primer sequences on each tag oligonucleotide sequence. In some disclosure herein the primer can be universal primers or unique primer sequences. Amplification allows for the preparation of the target nucleic acids for sequencing, as well as to retrieve the target nucleic acids having the desired sequences after sequencing. In some disclosure herein, a sample of the nucleic acid molecules is subjected to transposon-mediated fragmentation and adapter ligation to enable rapid preparation for paired end reads using high throughput sequencing systems.

One skilled in the art will appreciate that it can be important to control the extent of the fragmentation and the size of the nucleic acid fragments to maximize the number of reads in the sequencing paired reads and thereby to allow for sequencing the desired length of the disclosure herein fragment. In some disclosure herein the paired end reads can generate one sequence with a tag for identification, and another sequence which is internal to the construct target region. With high throughput sequencing, enough coverage can be generated to reconstruct the consensus sequence of each tag pair construct and determine if the construct sequence is correct. In some disclosure herein it is preferable to limit the number of breakage to less than 2, less than 3, or less than 4. In some disclosure herein the extent of the fragmentation and/or the size of the fragments can be controlled using appropriate reaction conditions such as by using the suitable concentration of transposon enzyme and controlling the temperature and time of incubation. Suitable reaction conditions can be obtained by using known amounts of a test library and titrating the enzyme and time to build a standard curve for actual sample libraries. In some disclosure herein a portion of the sample which is not used for fragmentation can be mixed back into the fragmented sample and processed for sequencing.

The sample can then be sequenced on a platform that generates paired end reads. Depending on the size of the individual DNA constructs, the number of constructs mixed together, and the estimated error rate of the populations, the appropriate platform can be chosen to maximize the number of reads desired and minimize the cost per construct.

The sequencing of the nucleic acid molecules results in reads with both of the tags from each molecule in the paired end reads. The paired end reads can be used to identify which pairs of tags were ligated or PCR joined and the identity of the molecule.

### Data Analysis

In some disclosure herein sequencing data or reads are analyzed. A read can represent consecutive base calls associated with a sequence of a nucleic acid. It should be understood that a read could include the full length sequence of the sample nucleic acid template or a portion thereof such as the sequence comprising the barcode sequence, the sequence identifier, and a portion of the target sequence. A read can comprise a small number of base calls, such as about eight nucleotides (base calls) but can contain larger numbers of base calls as well, such as 16 or more base calls, 25 or more base calls, 50 or more base calls, 100 or more base calls, or 200 or more nucleotides or base calls.

For data analysis, reads for which one tag is paired with multiple other tags for the same construct are discarded, because this would result in ambiguity as to which clone the data came from.

The sequencing results can then be analyzed to determine the sequences of each clone of each construct. For each paired read where one read contains a tag sequence, the identity of the molecule each sequencing read comes from is known, and the construct sequence itself can be used to distinguish between constructs with the same tag. The other read from the paired read can be used to build a consensus sequence of the internal regions of the molecule. From these results, a mapping of tag pairs corresponding to correct target sequence for each construct can be generated.

According to disclosure herein the analysis can comprise one or more of the following: (1) feature annotation; (2) feature correction; (3) identity assignment and confidence; (4) consensus call and confidence; and (5) preparative isolation.

Disclosure herein provides the ability to generate a consensus sequence for each nucleic acid construct. Each base called in a sequence can be based upon a consensus base call for that particular position based upon multiple reads at that position. These multiple reads are then assembled or compared to provide a consensus determination of a given base at a given position, and as a result, a consensus sequence for the particular sequence construct. It will be appreciated that any method of assigning a consensus determination to a particular base call from multiple reads of that position of sequence, are envisioned and encompassed by the disclosure herein. Methods for determining such call are known in the art. Such methods can include heuristic methods for multiple-sequence alignment, optimal methods for multiple sequences alignment, or any methods know in the art. In some disclosure herein, the sequence reads are aligned to a reference sequence (e.g. predetermined sequence of interest). High throughput sequencing requires efficient algorithms for mapping multiple query sequences such as short reads of the sequence identifiers or barcodes to such reference sequences.

### Isolation of target nucleic acid sequences

Disclosure herein is especially useful for isolating nucleic acid sequences of interest from a pool comprising nucleic acid sequences comprising sequences errors. The technology provided herein can embrace any method of non-destructive sequencing. Non-limiting examples of non-destructive sequencing include pyrosequencing, as originally described by Hyman et al., (1988, Anal. Biochem. 74: 324-436) and bead-based sequencing, described for instance by Leamon et al., (2004, Electrophoresis 24: 3769-3777). Non-destructive sequencing also includes methods using cleavable labeled oligonucleotides, as the above described Mitra et al., (2003, Anal. Biochem. 320:55-62) and photocleavable linkers (Seo et al., 2005, PNAS 102: 5926-5933). Methods using reversible terminators are also embraced by the technology provided herein (Metzker et al,. 1994, NAR 22: 4259-4267). Further methods for non-destructive sequencing (including single molecule sequencing) are described in US patents 7,133,782 and US 7,169,560.

Methods to selectively extract or isolate the correct sequence from the incorrect sequences are provided herein. The term "selective isolation", as used herein, can involve physical isolation of a desired nucleic acid molecule from others as by selective physical movement of the desired nucleic acid molecule, selective inactivation, destruction, release, or removal of other nucleic acid molecules than the nucleic acid molecule of interest. It should be appreciated that a nucleic acid molecule or library of nucleic acid constructs may include some errors that may result from sequence errors introduced during the oligonucleotides synthesis, the synthesis of the assembly nucleic acids and/or from assembly errors during the assembly reaction. Unwanted nucleic acids may be present in some disclosure herein. For example, between 0% and 50% (e.g., less than 45%, less than 40%, less than 35%, less than 30%, less than 25%, less than 20%, less than 15%, less than 10%, less than 5% or less than 1%) of the sequences in a library may be unwanted sequences.

In some disclosure herein the target having the desired sequence can be recovered using the methods for recovery of the annotated correct target sequences disclosed herein. In some disclosure herein the tag sequence pairs for each correct target sequence can be used to amplify by PCR the construct from the sample pool. It should be noted that since the likelihood of the same pair being used for multiple molecules is extremely low, the likelihood to isolate the nucleic acid molecule having the correct sequence is high. Yet in other disclosure herein the nucleic acid having the desired sequence can be recovered directly from the sequencer. In some disclosure herein the identity of a full length construct can be determined once the pairs of tags are identified. In principle, the location of the full length read (corresponding to a paired end read with the 5' and 3' tags) can be determined on the original sequencing flow cell. After locating the cluster on the flow cell surface, molecules can be eluted or otherwise captured from the surface.

In some disclosure herein nucleic acids can be sequenced in a sequencing channel. In some disclosure herein the nucleic acid constructs can be sequenced in situ on the solid support used in gene synthesis and reused/recycled therefrom. Analysis of the sequence information from the oligonucleotides permits the identification of those nucleic acid molecules that appear to have desirable sequences and those that do not. Such analysis of the sequence information can be qualitative, e.g., providing a positive or negative answer with regard to the presence of one or more sequences of interest (e.g., in stretches of 10 to 120 nucleotides). In some disclosure herein target nucleic acid molecules of interest can then be selectively isolated from the rest of the population. The sorting of individual nucleic acid molecules can be facilitated by the use of one or more solid supports (e.g. bead, insoluble polymeric material, planar surface, membrane, porous or non porous surface, chip, or any suitable support, etc...) to which the nucleic acid molecules can be immobilized. For example, the nucleic acid molecules can be immobilized on a porous surface such as a glass surface or a glass bead. Yet in other examples, the nucleic acid can be immobilized on a flow-through system such as a porous membrane or the like. Nucleic acid molecules determined to have the correct desired sequence can be selectively released or selectively copied.

If the nucleic acid molecules are located in different locations, e.g. in separate wells of a substrate, the nucleic acid molecules can be taken selectively from the wells identified as containing nucleic acid molecules with desirable sequences. For example, in the apparatus of Margulies et al., polony beads are located in individual wells of a fiber-optic slide. Physical extraction of the bead from the appropriate well of the apparatus permits the subsequent amplification or purification of the desirable nucleic acid molecules free of other contaminating nucleic acid molecules. Alternatively, if the nucleic acid molecules are attached to the beads using a selectively cleavable linker, cleavage of the linker (e.g., by increasing the pH in the well to cleave a base-labile linker) followed by extraction of the solvent in the well can be used to selectively isolate the nucleic acid molecules without physical manipulation of the bead. Likewise, if the method of Shendure et al. is used, physical extraction of the beads or of the portions of the gel containing the nucleic acid molecules of interest can be used to selectively isolate desired nucleic acid molecules.

Certain other methods of selective isolation involve the targeting of nucleic acid molecules without a requirement for physical manipulation of a solid support. Such methods can incorporate the use of an optical system to specifically target radiation to individual nucleic acid disclosure herein molecules. In some disclosure herein destructive radiation can be selectively targeted against undesired nucleic acid molecules (e.g., using micromirror technology) to destroy or disable them, leaving a population enriched for desired nucleic acid molecules. This enriched population can then be released from solid support and/or amplified, e.g., by PCR.

Example of methods and systems for selectively isolating the desired product (e.g. nucleic acids of interest) can use a laser tweezer or optical tweezer. Laser tweezers have been used for approximately two decades in the fields of biotechnology, medicine and molecular biology to position and manipulate micrometer-sized and submicrometer-sized particles (A. Ashkin, Science, (210), pp 1081-1088, 1980). By focusing the laser beam on the desired location (e.g. bead, well etc...) comprising the desired nucleic acid molecule of interest, the desired vessel remain optically trapped while the undesired nucleic acid sequences are eluted. Once all of the undesirable materials are washed off, the optical tweezer can be tuned off allowing the release the desired nucleic acid molecules.

Another method to capture the desirable products is by ablating the undesirable nucleic acids. In some disclosure herein a high power laser can be used to generate enough energy to disable, degrade, or destroy the nucleic acid molecules in areas where undesirable materials exist. The area where desirable nucleic acids exist does not receive any destructive energy, hence preserving its contents.

In some disclosure herein error-containing nucleic acid constructs can be eliminated. According to some disclosure herein the method comprises generating a nucleic acid having oligonucleotide tags at its 5' end and 3' end. For example, after assembly of the target sequences (e.g. full length nucleic acid constructs), the target sequences can be barcoded or alternatively, the target sequence can be assembled from a plurality of oligonucleotides designed such that the target sequence has a barcode at its 5' end and its 3' end. The tagged target sequence can be fragmented and sequenced using, for example, next-generation sequencing as provided herein. After identification of error-free target sequences, error-free target sequences can be recovered from directly from the next-generation sequencing plate. In some disclosure herein error-containing nucleic acids can be eliminated using laser ablation or any suitable method capable of eliminating undesired nucleic acid sequences. The error-free nucleic acid sequences can be eluted from the sequencing plate. Eluted nucleic acid sequences can be amplified using primers that are specific to the target sequences.

In some disclosure herein, the target polynucleotides can be amplified after obtaining clonal populations. In some disclosure herein, the target polynucleotide may comprise universal (common to all oligonucleotides), semi-universal (common to at least a portion of the oligonucleotides) or individual or unique primer (specific to each oligonucleotide) binding sites on either the 5' end or the 3' end or both. As used herein, the term "universal" primer or primer binding site means that a sequence used to amplify the oligonucleotide is common to all oligonucleotides such that all such oligonucleotides can be amplified using a single set of universal primers. In other circumstances, an oligonucleotide contains a unique primer binding site. As used herein, the term "unique primer binding site" refers to a set of primer recognition sequences that selectively amplifies a subset of oligonucleotides. In yet other circumstances, a target nucleic acid molecule contains both universal and unique amplification sequences, which can optionally be used sequentially.

In some disclosure herein, a binding tag capable of binding error-free nucleic acid molecules or a solid support comprising a binding tag can be added to the error-free nucleic acid sequences. For example, the binding tag, solid support comprising binding tag or solid support capable of binding nucleic acid can be added to locations of the sequencing plate or flow cells identified to include error-free nucleic acid sequences. In some disclosure herein the binding tag has a sequence complementary to the target nucleic acid sequence. In some disclosure herein the binding tag is a double-stranded sequence designed for either hybridization or ligation capture of nucleic acid of interest.

In some disclosure herein the solid support can be a bead. In some disclosure herein the bead can be disposed onto a substrate. The beads can be disposed on the substrate in a number of ways. Beads, or particles, can be deposited on a surface of a substrate such as a well or flow cell and can be exposed to various reagents and conditions which permit detection of the tag or label. In some disclosure herein the binding tags or beads can be deposited by inkjet at specific location of a sequencing plate.

In some disclosure herein beads can be derivatized in-situ with binding tags that are complementary to the barcodes or the additional sequences appended to the nucleic acids to capture, and/or enrich, and/or amplify the target nucleic acids identified to have the correct nucleic acid sequences (e.g. error-free nucleic acid). Nucleic acids can be immobilized on the beads by hybridization, covalent attachment, magnetic attachment, affinity attachment and the like. Hybridization is usually performed under stringent conditions. In some disclosure herein the binding tags can be universal or generic primers complementary to non-target sequences, for example all barcodes or to appended additional sequences. In some disclosure herein each bead can have binding tags capable of binding sequences present both the 5' end and the 3' end of the target molecules. Upon binding the target molecules, a loop-like structure is produced. Yet in other disclosure herein beads can have a binding tag capable of binding sequences present at the 3' end of the target molecule. Yet in other disclosure herein beads can have a binding tag capable of binding sequences present at the 5' end of the target molecule.

Beads, such as magnetic or paramagnetic beads, can be added to the each well or arrayed on a solid support. For example, Solid Phase Reversible Immobilization (SPRI) beads from Beckman Coulter can be used. In some disclosure herein the pool of constructs can be distributed to the individual wells containing the beads. Additional thermal cycling can be used to enhance capture specificity. Using standard magnetic capture, the solution can then be removed followed by subsequent washing of the conjugated beads Amplification of the desired construct clone can be done either on bead or after release of the captured clone. In some disclosure herein the beads can be configured for either hybridization or ligation based capture using double-stranded sequences on the bead.

A variation of the bead-based process can involve a set of flow-sortable encoded beads. Bead-based methods can employ nucleic acid hybridization to a capture probe or attachment on the surface of distinct populations of capture beads. Such encoded beads can be used on a pool of constructs and then sorted into individual wells for downstream amplification, isolation and clean up. While the use of magnetic beads described above can be particularly useful, other methods to separate beads can be envisioned in some disclosure herein. The capture beads may be labeled with a fluorescent moiety which would make the target-capture bead complex fluorescent. For example, the beads can be impregnated with a fluorophore thereby creating distinct populations of beads that can be sorted according to the fluorescence wavelength. The target capture bead complex may be separated by flow cytometry or fluorescence cell sorter. In some disclosure herein the beads can vary is size, or in any suitable characteristics allowing the sorting of distinct population of beads. For example, using capture beads having distinct sizes would allow separation by filtering or other particle size separation techniques.

In some disclosure herein the flow-sortable encoded beads can be used to isolate the nucleic acid constructs prior to or after post-synthesis release. Such process allows for sorting by construct size, customer etc.

In some disclosure herein primers can be loaded onto generic beads, for example, magnetic beads. Each bead can be derivatized many times to have many primers bound to it. In some disclosure herein derivatization allows to have two or more different primers bound per bead, or to have the same primer bound per bead. Such beads can be distributed in each well of a multi-well plate. Beads can be loaded with barcodes capable of capturing specific nucleic acid molecules, for example by hybridizing a nucleic acid sequence comprising the barcode and a sequence complementary to the primer(s) loaded onto the generic beads. The sample comprising the double-stranded pooled nucleic acids can be subjected to appropriate conditions to render the double-stranded nucleic acids single-stranded. For example, the double-stranded nucleic acids can be subjected to any denaturation conditions known in the art. The pooled single-stranded sample can be distributed across all the wells of a multi-well plate. Under appropriate conditions, the derivatized beads comprising the barcodes can capture specific nucleic acid molecules in each well, based on the exact barcodes (5' and 3') loaded onto the beads in each well. The beads can then be washed. For example, when using magnetic beads, the beads can be pulled down with a magnet, allowing washing and removal of the solution. In some disclosure herein the beads can be washed iteratively. The nucleic acids that remained bound on the beads can then amplified using PCR to produce individual clones in each well of the multi-well construct plate.

In some aspects of the invention, nanopore sequencing can be used to sequence individual nucleic acid strand at single nucleotide level. One of skill in the art would appreciate that nanopore sequencing has the advantage of minimal sample preparation, sequence readout that does not require nucleotides, polymerases or ligases, and the potential of very long read-lengths. However, nanopore sequencing can have relatively high error rates (∼ 10% error per base). In some disclosure herein the nanopore sequencing device comprises a shuntable microfluidic flow valve to recycle the full length nucleic acid construct so as to allow for multiple sequencing passes. In some disclosure herein the nanopores can be connected in series with a shuntable microfluidic flow valve such that full length nucleic acid construct can be shunted back to the nanopore several times to allow for multiple sequencing passes. Using these configurations, the full length nucleic acid molecules can be sequenced two or more times. Resulting error-free nucleic acid sequences may be shunted to a collection well for recovery and use.

In some disclosure herein, alternative preparative sequencing methods are provided herein. The methods comprise circularizing the target nucleic acid (e.g. the full length target nucleic acid) using double-ended primers capable of binding the 5' end and the 3' end of the target nucleic acids. In some disclosure herein the double-ended primers have sequences complementary to the 5' end and the 3' end barcodes. Nucleases can be added so as to degrade the linear nucleic acid, thus locking-in the desired constructs. Optionally, the target nucleic acid can be amplified using primers specific to the target nucleic acids.

### Synthetic oligonucleotides

Synthetic oligonucleotides can be generated using standard DNA synthesis chemistry (e.g. phosphoramidite method). Synthetic oligonucleotides may be synthesized on a solid support, such as for example a microarray, using any appropriate technique known in the art. Oligonucleotides can be eluted from the microarray prior to be subjected to amplification or can be amplified on the microarray.

As used herein, an oligonucleotide may be a nucleic acid molecule comprising at least two covalently bonded nucleotide residues. In some disclosure herein an oligonucleotide may be between 10 and 1,000 nucleotides long. For example, an oligonucleotide may be between 10 and 500 nucleotides long, or between 500 and 1,000 nucleotides long. In some disclosure herein an oligonucleotide may be between about 20 and about 300 nucleotides long (e.g., from about 30 to 250, from about 40 to 220 nucleotides long, from about 50 to 200 nucleotides long, from about 60 to 180 nucleotides long, or from about 65 or about 150 nucleotides long), between about 100 and about 200 nucleotides long, between about 200 and about 300 nucleotides long, between about 300 and about 400 nucleotides long, or between about 400 and about 500 nucleotides long. However, shorter or longer oligonucleotides may be used. An oligonucleotide may be a single-stranded or double-stranded nucleic acid. As used herein the terms "nucleic acid", "polynucleotide", "oligonucleotide" are used interchangeably and refer to naturally-occurring or synthetic polymeric forms of nucleotides. The oligonucleotides and nucleic acid molecules of the present disclosure may be formed from naturally occurring nucleotides, for example forming deoxyribonucleic acid (DNA) or ribonucleic acid (RNA) molecules. Alternatively, the naturally occurring oligonucleotides may include structural modifications to alter their properties, such as in peptide nucleic acids (PNA) or in locked nucleic acids (LNA), or other position modifications. The solid phase synthesis of oligonucleotides and nucleic acid molecules with naturally occurring or artificial bases is well known in the art. The terms should be understood to include equivalents, analogs of either RNA or DNA made from nucleotide analogs and as applicable to the disclosure being described, single-stranded or double-stranded polynucleotides. Nucleotides useful in the disclosure include, for example, naturally-occurring nucleotides (for example, ribonucleotides or deoxyribonucleotides), or natural or synthetic modifications of nucleotides, or artificial bases. As used herein, the term monomer refers to a member of a set of small molecules which are and can be joined together to form an oligomer, a polymer or a compound composed of two or more members. The particular ordering of monomers within a polymer is referred to herein as the "sequence" of the polymer. The set of monomers includes but is not limited to example, the set of common L-amino acids, the set of D- amino acids, the set of synthetic and/or natural amino acids, the set of nucleotides and the set of pentoses and hexoses. Disclosure herein described herein primarily with regard to the preparation of oligonucleotides, but could readily be applied in the preparation of other polymers such as peptides or polypeptides, polysaccharides, phospholipids, heteropolymers, polyesters, polycarbonates, polyureas, polyamides, polyethyleneimines, polyarylene sulfides, polysiloxanes, polyimides, polyacetates, or any other polymers.

In some disclosure herein the methods and devices provided herein can use oligonucleotides that are immobilized on a surface or substrate (e.g., support-bound oligonucleotides) where either the 3' or 5' end of the oligonucleotide is bound to the surface. Support-bound oligonucleotides comprise for example, oligonucleotides complementary to construction oligonucleotides, anchor oligonucleotides and/or spacer oligonucleotides. As used herein the term "support", "substrate" and "surface" are used interchangeably and refers to a porous or non-porous solvent insoluble material on which polymers such as nucleic acids are synthesized or immobilized. As used herein "porous" means that the material contains pores having substantially uniform diameters (for example in the nm range). Porous materials include paper, synthetic filters, polymeric matrices, etc. In such porous materials, the reaction may take place within the pores or matrix. The support can have any one of a number of shapes, such as pin, strip, plate, disk, rod, bends, cylindrical structure, particle, including bead, nanoparticles and the like. The support can have variable widths. The support can be hydrophilic or capable of being rendered hydrophilic. The support can include inorganic powders such as silica, magnesium sulfate, and alumina; natural polymeric materials, particularly cellulosic materials and materials derived from cellulose, such as fiber containing papers, e.g., filter paper, chromatographic paper, etc.; synthetic or modified naturally occurring polymers, such as nitrocellulose, cellulose acetate, poly (vinyl chloride), polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), polyvinylidene difluoride (PVDF) membrane, glass, controlled pore glass, magnetic controlled pore glass, ceramics, metals, and the like etc.; either used by themselves or in conjunction with other materials. In some disclosure herein oligonucleotides are synthesized on an array format. For example, single-stranded oligonucleotides are synthesized in situ on a common support wherein each oligonucleotide is synthesized on a separate or discrete feature (or spot) on the substrate. In some disclosure herein single-stranded oligonucleotides can be bound to the surface of the support or feature. As used herein the term "array" refers to an arrangement of discrete features for storing, amplifying and releasing oligonucleotides or complementary oligonucleotides for further reactions. In some disclosure herein the support or array is addressable: the support includes two or more discrete addressable features at a particular predetermined location (i.e., an "address") on the support. Therefore, each oligonucleotide molecule of the array is localized to a known and defined location on the support. The sequence of each oligonucleotide can be determined from its position on the support.

In some disclosure herein oligonucleotides are attached, spotted, immobilized, surface-bound, supported or synthesized on the discrete features of the surface or array. Oligonucleotides may be covalently attached to the surface or deposited on the surface. Arrays may be constructed, custom ordered or purchased from a commercial vendor (e.g., Agilent, Affymetrix, Nimblegen). Various methods of construction are well known in the art e.g., maskless array synthesizers, light directed methods utilizing masks, flow channel methods, spotting methods, etc. In some disclosure herein construction and/or selection oligonucleotides may be synthesized on a solid support using maskless array synthesizer (MAS).

### Applications

Disclosure herein may be useful for a range of applications involving the production and/or use of synthetic nucleic acids. As described herein, the disclosure herein provides methods for producing synthetic nucleic acids having the desired sequence with increased efficiency. The resulting nucleic acids may be amplified in vitro (e.g., using PCR, LCR, or any suitable amplification technique), amplified in vivo (e.g., via cloning into a suitable vector), isolated and/or purified. An assembled nucleic acid (alone or cloned into a vector) may be transformed into a host cell (e.g., a prokaryotic, eukaryotic, insect, mammalian, or other host cell). In some disclosure herein the host cell may be used to propagate the nucleic acid. In certain disclosure herein the nucleic acid may be integrated into the genome of the host cell. In some disclosure herein the nucleic acid may replace a corresponding nucleic acid region on the genome of the cell (e.g., via homologous recombination). Accordingly, nucleic acids may be used to produce recombinant organisms. In some disclosure herein a target nucleic acid may be an entire genome or large fragments of a genome that are used to replace all or part of the genome of a host organism. Recombinant organisms also may be used for a variety of research, industrial, agricultural, and/or medical applications.

Many of the techniques described herein can be used together, applying suitable assembly techniques at one or more points to produce long nucleic acid molecules. For example, ligase-based assembly may be used to assemble oligonucleotide duplexes and nucleic acid fragments of less than 100 to more than 10,000 base pairs in length (e.g., 100 mers to 500 mers, 500 mers to 1,000 mers, 1,000 mers to 5,000 mers, 5, 000 mers to 10,000 mers, 25,000 mers, 50,000 mers, 75,000 mers, 100,000 mers, etc.). In an exemplary disclosure herein methods described herein may be used during the assembly of an entire genome (or a large fragment thereof, e.g., about 10%, 20%, 30%, 40%, 50%, 60%, 70%, 80%, 90%, or more) of an organism (e.g., of a viral, bacterial, yeast, or other prokaryotic or eukaryotic organism), optionally incorporating specific modifications into the sequence at one or more desired locations.

Any of the nucleic acid products (e.g., including nucleic acids that are amplified, cloned, purified, isolated, etc.) may be packaged in any suitable format (e.g., in a stable buffer, lyophilized, etc.) for storage and/or shipping (e.g., for shipping to a distribution center or to a customer). Similarly, any of the host cells (e.g., cells transformed with a vector or having a modified genome) may be prepared in a suitable buffer for storage and or transport (e.g., for distribution to a customer). In some disclosure herein cells may be frozen. However, other stable cell preparations also may be used.

Host cells may be grown and expanded in culture. Host cells may be used for expressing one or more RNAs or polypeptides of interest (e.g., therapeutic, industrial, agricultural, and/or medical proteins). The expressed polypeptides may be natural polypeptides or non-natural polypeptides. The polypeptides may be isolated or purified for subsequent use.

Accordingly, nucleic acid molecules generated using methods of the disclosure herein can be incorporated into a vector. The vector may be a cloning vector or an expression vector. In some disclosure herein the vector may be a viral vector. A viral vector may comprise nucleic acid sequences capable of infecting target cells. Similarly, in some disclosure herein a prokaryotic expression vector operably linked to an appropriate promoter system can be used to transform target cells. In other disclosure herein a eukaryotic vector operably linked to an appropriate promoter system can be used to transfect target cells or tissues.

Transcription and/or translation of the constructs described herein may be carried out in vitro (i.e. using cell-free systems) or in vivo (i.e. expressed in cells). In some disclosure herein cell lysates may be prepared. In certain disclosure herein expressed RNAs or polypeptides may be isolated or purified. Nucleic acids of the disclosure also may be used to add detection and/or purification tags to expressed polypeptides or fragments thereof. Examples of polypeptide-based fusion/tag include, but are not limited to, hexa-histidine (His⁶) Myc and HA, and other polypeptides with utility, such as GFP₅ GST, MBP, chitin and the like. In some disclosure herein polypeptides may comprise one or more unnatural amino acid residue(s).

In some disclosure herein antibodies can be made against polypeptides or fragment(s) thereof encoded by one or more synthetic nucleic acids. In certain disclosure herein synthetic nucleic acids may be provided as libraries for screening in research and development (e.g., to identify potential therapeutic proteins or peptides, to identify potential protein targets for drug development, etc.) In some disclosure herein a synthetic nucleic acid may be used as a therapeutic (e.g., for gene therapy, or for gene regulation). For example, a synthetic nucleic acid may be administered to a patient in an amount sufficient to express a therapeutic amount of a protein. In other disclosure herein, a synthetic nucleic acid may be administered to a patient in an amount sufficient to regulate (e.g., down-regulate) the expression of a gene.

It should be appreciated that different acts or embodiments described herein may be performed independently and may be performed at different locations in the United States or outside the United States. For example, each of the acts of receiving an order for a target nucleic acid, analyzing a target nucleic acid sequence, designing one or more starting nucleic acids (e.g., oligonucleotides), synthesizing starting nucleic acid(s), purifying starting nucleic acid(s), assembling starting nucleic acid(s), isolating assembled nucleic acid(s), confirming the sequence of assembled nucleic acid(s), manipulating assembled nucleic acid(s) (e.g., amplifying, cloning, inserting into a host genome, etc.), and any other acts or any parts of these acts may be performed independently either at one location or at different sites within the United States or outside the United States. In some embodiments, an assembly procedure may involve a combination of acts that are performed at one site (in the United States or outside the United States) and acts that are performed at one or more remote sites (within the United States or outside the United States).

### Automated applications

Aspects of the methods and devices provided herein may include automating one disclosure herein or more acts described herein. In some disclosure herein one or more steps of an amplification and/or assembly reaction may be automated using one or more automated sample handling devices (e.g., one or more automated liquid or fluid handling devices). Automated devices and procedures may be used to deliver reaction reagents, including one or more of the following: starting nucleic acids, buffers, enzymes (e.g., one or more ligases and/or polymerases), nucleotides, salts, and any other suitable agents such as stabilizing agents. Automated devices and procedures also may be used to control the reaction conditions. For example, an automated thermal cycler may be used to control reaction temperatures and any temperature cycles that may be used. In some disclosure herein a scanning laser may be automated to provide one or more reaction temperatures or temperature cycles suitable for incubating polynucleotides. Similarly, subsequent analysis of assembled polynucleotide products may be automated. For example, sequencing may be automated using a sequencing device and automated sequencing protocols. Additional steps (e.g., amplification, cloning, etc.) also may be automated using one or more appropriate devices and related protocols. It should be appreciated that one or more of the device or device components described herein may be combined in a system (e.g., a robotic system) or in a micro-environment (e.g., a micro-fluidic reaction chamber). Assembly reaction mixtures (e.g., liquid reaction samples) may be transferred from one component of the system to another using automated devices and procedures (e.g., robotic manipulation and/or transfer of samples and/or sample containers, including automated pipetting devices, micro-systems, etc.). The system and any components thereof may be controlled by a control system.

Accordingly, method steps and/or aspects of the devices provided herein may be automated using, for example, a computer system (e.g., a computer controlled system). A computer system on which aspects of the technology provided herein can be implemented may include a computer for any type of processing (e.g., sequence analysis and/or automated device control as described herein). However, it should be appreciated that certain processing steps may be provided by one or more of the automated devices that are part of the assembly system. In some disclosure herein a computer system may include two or more computers. For example, one computer may be coupled, via a network, to a second computer. One computer may perform sequence analysis. The second computer may control one or more of the automated synthesis and assembly devices in the system. In other aspects, additional computers may be included in the network to control one or more of the analysis or processing acts. Each computer may include a memory and processor. The computers can take any form, as the aspects of the technology provided herein are not limited to being implemented on any particular computer platform. Similarly, the network can take any form, including a private network or a public network (e.g., the Internet). Display devices can be associated with one or more of the devices and computers. Alternatively, or in addition, a display device may be located at a remote site and connected for displaying the output of an analysis in accordance with the technology provided herein. Connections between the different components of the system may be via wire, optical fiber, wireless transmission, satellite transmission, any other suitable transmission, or any combination of two or more of the above.

Each of the different disclosures of the technology provided herein can be independently automated and implemented in any of numerous ways. For example, each disclosure herein can be independently implemented using hardware, software or a combination thereof. When implemented in software, the software code can be executed on any suitable processor or collection of processors, whether provided in a single computer or distributed among multiple computers. It should be appreciated that any component or collection of components that perform the functions described above can be generically considered as one or more controllers that control the above-discussed functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation of the technology provided herein comprises at least one computer-readable medium (e.g., computer memory, flash memory, compact disk, etc.) encoded with a computer program (i.e., a plurality of instructions), which, when executed on a processor, performs one or more of the above-discussed functions of the technology provided herein. The computer-readable medium can be transportable such that the program stored thereon can be loaded onto any computer system resource to implement one or more functions of the technology provided herein. In addition, it should be appreciated that the reference to a computer program which, when executed, performs the above-discussed functions, is not limited to an application program running on a host computer. Rather, the term computer program is used herein in a generic sense to reference any type of computer code (e.g., software or microcode) that can be employed to program a processor to implement the above-discussed aspects of the technology provided herein.

It should be appreciated that in accordance with the technology provided herein wherein processes are stored in a computer readable medium, the computer-implemented processes may, during the course of their execution, receive input manually (e.g., from a user).

Accordingly, overall system-level control of the assembly devices or components described herein may be performed by a system controller which may provide control signals to the associated nucleic acid synthesizers, liquid handling devices, thermal cyclers, sequencing devices, associated robotic components, as well as other suitable systems for performing the desired input/output or other control functions. Thus, the system controller along with any device controllers together form a controller that controls the operation of a nucleic acid assembly system. The controller may include a general purpose data processing system, which can be a general purpose computer, or network of general purpose computers, and other associated devices, including communications devices, modems, and/or other circuitry or components to perform the desired input/output or other functions. The controller can also be implemented, at least in part, as a single special purpose integrated circuit (e.g., ASIC) or an array of ASICs, each having a main or central processor section for overall, system-level control, and separate sections dedicated to performing various different specific computations, functions and other processes under the control of the central processor section. The controller can also be implemented using a plurality of separate dedicated programmable integrated or other electronic circuits or devices, e.g., hard wired electronic or logic circuits such as discrete element circuits or programmable logic devices. The controller can also include any other components or devices, such as user input/output devices (monitors, displays, printers, a keyboard, a user pointing device, touch screen, or other user interface, etc.), data storage devices, drive motors, linkages, valve controllers, robotic devices, vacuum and other pumps, pressure sensors, detectors, power supplies, pulse sources, communication devices or other electronic circuitry or components, and so on. The controller also may control operation of other portions of a system, such as automated client order processing, quality control, packaging, shipping, billing, etc., to perform other suitable functions known in the art but not described in detail herein.

Various aspects of the present invention may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

The following examples are set forth as being representative of the present invention.

## Claims

1. A method for preparing nucleic acid molecules, the method comprising:
(a) providing transpososomes and a pool of different synthetic nucleic acid molecules having a length of 1 kbase or more, each synthetic nucleic acid molecule having a unique target nucleic acid sequence, each transpososome having at least one unique double-stranded oligonucleotide barcode;
(b) contacting the transpososomes and synthetic nucleic acid molecules under conditions sufficient to generate one or more double-stranded nucleic acid junction breaks, wherein each transpososome introduces separate correlated barcodes upstream and downstream of the junction break, thereby generating a plurality of nucleic acid fragments comprising a barcode at the 5' end and the 3' end; and
(c) determining the sequence of a barcoded nucleic acid fragment.

2. The method of claim 1 wherein in the step of contacting,
the one or more nucleic acid junction breaks are generated by cleaving the nucleic acid molecules,
wherein the transpososome introduces a unique double-stranded oligonucleotide sequence comprising two correlated barcodes separated by one or more cleavage sites to the nucleic acid molecules; wherein preferably the cleavage sites are restriction sites or one or more dU bases.

3. The method of any one of claims 1-2 wherein in the step of providing,
the 5' end and the 3' end of each of the target nucleic acid molecules are tagged with a unique nucleotide tag.

4. The method of any one of claims 1-3 and 8 wherein the step of determining comprises producing a sequence read using a next generation sequencing platform, wherein preferably the nucleic acid molecules have a length greater than a sequence read length limit Lmax imposed by the next generation sequencing platform, wherein more preferably each fragment has on average a finite probability of being less than the Lmax.

5. The method of any one of claims 1-4 wherein, in the step of contacting, a plurality of junction breaks are generated and each side of the junction breaks are tagged with correlated barcodes identifying an upstream and downstream side of the junction.

6. The method of any one of claims 1-5 further comprising amplifying the nucleic acid fragments.

7. The method of any one of claims 1-6 wherein the pool of nucleic acid molecules comprises error-free and error-containing nucleic acid molecules; wherein preferably the method further comprises amplifying error-free nucleic acid molecules having a predetermined sequence using primers having a sequence complementary to a sequence of the 5' end and the 3' end oligonucleotide tags; wherein more preferably the method further comprises isolating the error-free nucleic acid molecules having the predetermined sequence.

8. A method for preparing nucleic acid molecules, the method comprising:
(a) providing a pool of different synthetic nucleic acid molecules having a length of 1 kbase or more, each synthetic nucleic acid molecules having a unique target nucleic acid sequence, wherein each target nucleic acid sequence has an oligonucleotide tag sequence at the 5' end and the 3' end, and wherein the oligonucleotide tag sequence comprises a unique nucleotide tag;
(b) subjecting the synthetic nucleic acid molecules to fragmentation using transpososomes to generate nucleic acid fragments, wherein each nucleic acid fragment comprises a barcode sequence at the the 5' end and the 3' end; and
(c) determining the sequence of the tagged nucleic acid fragments.

9. The method of claim 8 wherein the pool of synthetic nucleic acid molecules comprises error-free and error-containing nucleic acid molecules.

10. The method of claim 8 or 9 wherein, in the step of subjecting, a plurality of junction breaks are generated and each side of junction breaks are tagged with correlated oligonucleotide barcodes identifying an upstream and downstream side of the junction break.

11. A method for preparing nucleic acid molecules, the method comprising:
(a) providing a pool of synthetic nucleic acid molecules having a length of 1 kbase or more comprising at least two different nucleic acid molecules, the pool of nucleic acid molecules comprising error-free and error-containing nucleic acid molecules and wherein each population of nucleic acid molecule has a unique target nucleic acid sequence having a 5' end and a 3' end;
(b) tagging the 5' end and the 3' end of the target nucleic acid molecules with an oligonucleotide tag sequence, wherein the oligonucleotide tag sequence comprises a unique nucleotide tag, thereby forming tagged target nucleic acid molecules;
(c) diluting the tagged target nucleic acid molecules to generate a pool of diluted tagged target molecules comprising at least one error-free tagged target nucleic acid molecule;
(d) providing transpososomes, wherein each transpososome has a different unique double-stranded oligonucleotide barcode;
(e) adding the transpososomes to the pool of tagged nucleic acid molecules;
(f) allowing the transpososomes to generate double-stranded nucleic acid junction breaks thereby generating a plurality of nucleic acid fragments comprising a barcode or an oligonucleotide tag sequence at the 5' end and at the 3' end; and
(g) determining the sequence of the tagged nucleic acid fragments.

12. The method of claim 11 wherein following the diluting step, the tagged target nucleic acid molecules are amplified.

13. The method of claim 12 further comprising diluting the amplified tagged target nucleic acid molecules.

14. The method of any one of claims 11-13 further comprising amplifying the plurality of tagged nucleic acid fragments.

15. The method of any one of claims 11-14 further comprising isolating the error-free nucleic acid molecules having the predetermined sequence.

## Patentansprüche

1. Verfahren zur Herstellung von Nucleinsäuremolekülen, wobei das Verfahren Folgendes umfasst:
(a) Bereitstellen von Transpososomen und einem Pool von unterschiedlichen synthetischen Nucleinsäuremolekülen mit einer Länge von 1 kbase oder mehr, wobei jedes synthetische Nucleinsäuremolekül eine einzigartige Ziel-Nucleinsäuresequenz aufweist und jedes Transpososom zumindest einen einzigartigen doppelsträngigen Oligonucleotid-Barcode aufweist;
(b) Kontaktieren der Transpososome und synthetischen Nucleinsäuremoleküle unter Bedingungen, die ausreichen, um einen oder mehrere Doppelstrang-Nucleinsäure-Bindungsbruch/brüche zu erzeugen, wobei jedes Transpososom getrennte, korrelierende Barcodes stromauf und stromab der Bindungsbrüche einführt, wodurch eine Vielzahl von Nucleinsäurefragmenten erzeugt wird, die einen Barcode am 5'-Ende und am 3'-Ende umfassen; und
(c) Bestimmen der Sequenz eines mit Barcode versehenen Nucleinsäurefragments.

2. Verfahren nach Anspruch 1, wobei bei dem Schritt des Kontaktierens
der eine oder die mehreren Nucleinsäure-Bindungsbruch/brüche erzeugt wird/werden, indem die Nucleinsäuremoleküle gespalten werden,
wobei das Transpososom eine einzigartige doppelsträngige Oligonucleotidsequenz einführt, die zwei korrelierende Barcodes umfasst, die durch eine oder mehrere Spaltungsstelle(n) von den Nucleinsäuremolekülen getrennt sind; wobei die Spaltungsstellen vorzugsweise Restriktionsstellen oder eine oder mehrere dU-Base(n) sind.

3. Verfahren nach einem der Ansprüche 1 bis 2, wobei im Schritt des Bereitstellens
das 5'-Ende und das 3'-Ende jedes der Ziel-Nucleinsäuremoleküle mit einer einzigartigen Nucleotid-Markierung markiert werden.

4. Verfahren nach einem der Ansprüche 1 bis 3 und 8, wobei der Schritt des Bestimmens das Erstellen einer Sequenz-Auslesung unter Verwendung einer Next-Generation-Sequenzierplattform umfasst, wobei die Nucleinsäuremoleküle vorzugsweise eine größere Länge aufweisen als ein Sequenz-Auslesungslänge-Limit Lmax, das von der Next-Generation-Sequenzierplattform vorgegeben wird, wobei noch bevorzugter jedes Fragment durchschnittlich eine endliche Wahrscheinlichkeit aufweist, kürzer als Lmax zu sein.

5. Verfahren nach einem der Ansprüche 1 bis 4, wobei im Schritt des Kontaktierens eine Vielzahl von Bindungsbrüchen erzeugt werden und beide Seiten der Bindungsbrüche mit korrelierenden Barcodes markiert sind, die eine Stromauf- und eine Stromab-Seite der Bindung identifizieren.

6. Verfahren nach einem der Ansprüche 1 bis 5, das weiters die Amplifikation der Nucleinsäurefragmente umfasst.

7. Verfahren nach einem der Ansprüche 1 bis 6, wobei der Pool von Nucleinsäuremolekülen fehlerfreie und fehlerhältige Nucleinsäuremoleküle umfasst; wobei das Verfahren vorzugsweise weiters das Amplifizieren von fehlerfreien Nucleinsäuremolekülen mit einer vorbestimmten Sequenz unter Verwendung von Primern mit einer Sequenz, die komplementär zu einer Sequenz der 5'-End- und der 3'-End-Oligonucleotidmarkierungen ist, umfasst; wobei das Verfahren noch bevorzugter weiters das Isolieren der fehlerfreien Nucleinsäuremoleküle mit der vorbestimmten Sequenz umfasst.

8. Verfahren zur Herstellung von Nucleinsäuremolekülen, wobei das Verfahren Folgendes umfasst:
(a) Bereitstellen eines Pools von unterschiedlichen synthetischen Nucleinsäuremolekülen mit einer Länge von 1 kbase oder mehr, wobei jedes synthetische Nucleinsäuremolekül eine einzigartige Ziel-Nucleinsäuresequenz aufweist, wobei jede Ziel-Nucleinsäuresequenz eine Oligonucleotid-Markierungssequenz am 5'-Ende und am 3'-Ende aufweist und wobei die Oligonucleotid-Markierungssequenz eine einzigartige Nucleotid-Markierung umfasst;
(b) Unterziehen der synthetischen Nucleinsäuremoleküle einer Fragmentierung unter Verwendung von Transpososomen, um Nucleinsäurefragmente zu erzeugen, wobei jedes Nucleinsäurefragment eine Barcode-Sequenz am 5'-Ende und am 3'-Ende umfasst; und
(c) Bestimmen der Sequenz der markierten Nucleinsäurefragmente.

9. Verfahren nach Anspruch 8, wobei der Pool von synthetischen Nucleinsäuremolekülen fehlerfreie und fehlerhältige Nucleinsäuremoleküle umfasst.

10. Verfahren nach Anspruch 8 oder 9, wobei im Schritt des Unterziehens eine Vielzahl von Bindungsbrüchen erzeugt wird und jede Seite der Bindungsbrüche mit korrelierenden Oligonucleotid-Barcodes markiert ist, die eine Stromauf- und eine Stromab-Seite des Bindungsbruchs identifizieren.

11. Verfahren zur Herstellung von Nucleinsäuremolekülen, wobei das Verfahren Folgendes umfasst:
(a) Bereitstellen eines Pools von synthetischen Nucleinsäuremolekülen mit einer Länge von 1 kbase oder mehr, der zumindest zwei unterschiedliche Nucleinsäuremoleküle umfasst, wobei der Pool von Nucleinsäuremolekülen fehlerfreie und fehlerhältige Nucleinsäuremoleküle umfasst und wobei jede Population von Nucleinsäuremolekülen eine einzigartige Ziel-Nucleinsäuresequenz mit einem 5'-Ende und einem 3'-Ende aufweist;
(b) Markieren des 5'-Endes und des 3'-Endes der Ziel-Nucleinsäuremoleküle mit einer Oligonucleotid-Markierungssequenz, wobei die Oligonucleotid-Markierungssequenz eine einzigartige Nucleotid-Markierung umfasst, wodurch markierte Ziel-Nucleinsäuremoleküle gebildet werden;
(c) Verdünnen der markierten Ziel-Nucleinsäuremoleküle, um einen Pool von verdünnten, markierten Ziel-Molekülen zu erzeugen, der zumindest ein fehlerfreies markiertes Ziel-Nucleinsäuremolekül umfasst;
(d) Bereitstellen von Transpososomen, wobei jedes Transpososom einen unterschiedlichen einzigartigen doppelsträngigen Oligonucleotid-Barcode aufweist;
(e) Zusetzen der Transpososome zum Pool von markierten Nucleinsäuremolekülen;
(f) den Transpososomen erlauben, Doppelstrang-Nucleinsäure-Bindungsbrüche zu erzeugen, wodurch eine Vielzahl von Nucleinsäurefragmenten, die einen Barcode oder eine Oligonucleotid-Markierungssequenz am 5'-Ende und am 3'-Ende umfassen, erzeugt wird; und
(g) Bestimmen der Sequenz der markierten Nucleinsäurefragmente.

12. Verfahren nach Anspruch 11, wobei nach dem Verdünnungsschritt die markierten Ziel-Nucleinsäuremoleküle amplifiziert werden.

13. Verfahren nach Anspruch 12, das weiters das Verdünnen der amplifizierten markierten Ziel-Nucleinsäuremoleküle umfasst.

14. Verfahren nach einem der Ansprüche 11 bis 13, das weiters das Amplifizieren der Vielzahl von markierten Nucleinsäurefragmenten umfasst.

15. Verfahren nach einem der Ansprüche 11 bis 14, das weiters das Isolieren der fehlerfreien Nucleinsäuremoleküle mit der vorbestimmten Sequenz umfasst.

## Revendications

1. Procédé pour préparer des molécules d'acide nucléique, le procédé comprenant :
(a) la mise à disposition de transpososomes et d'un pool de molécules d'acide nucléique synthétiques différentes possédant une longueur de 1 kbase ou plus, chaque molécule d'acide nucléique synthétique possédant une séquence d'acide nucléique cible unique, chaque transpososome possédant au moins un code-barres oligonucléotidique double brin unique ;
(b) la mise en contact des transpososomes et des molécules d'acide nucléique synthétiques dans des conditions suffisantes pour générer une ou plusieurs cassures de jonction d'acide nucléique double brin, où chaque transpososome introduit des codes-barres corrélés distincts en amont et en aval de la cassure de jonction, en générant ainsi une pluralité de fragments d'acide nucléique comprenant un code-barres à l'extrémité 5' et à l'extrémité 3' ; et
(c) la détermination de la séquence d'un fragment d'acide nucléique à code-barres.

2. Procédé selon la revendication 1, dans lequel, à l'étape de mise en contact,
la une ou plusieurs cassures de jonction d'acide nucléique sont générées en clivant les molécules d'acide nucléique,
dans lequel le transpososome introduit une séquence d'oligonucléotide double brin unique comprenant deux codes-barres corrélés séparés par un ou plusieurs sites de clivage dans les molécules d'acide nucléique ; dans lequel de préférence les sites de clivage sont des sites de restriction ou une ou plusieurs bases dU.

3. Procédé selon l'une quelconque des revendications 1 à 2, dans lequel, à l'étape de mise à disposition,
l'extrémité 5' et l'extrémité 3' de chacune des molécules d'acide nucléique cible sont marquées avec un marqueur nucléotidique unique.

4. Procédé selon l'une quelconque des revendications 1 à 3 et 8, dans lequel l'étape de détermination comprend la production d'une lecture de séquence en utilisant une plate-forme de séquençage de dernière génération, dans lequel de préférence les molécules d'acide nucléique possèdent une longueur supérieure à une limite de longueur de lecture de séquence Lmax imposée par la plate-forme de séquençage de dernière génération, dans lequel, de manière davantage préférée, chaque fragment possède en moyenne une probabilité finie d'être inférieur à la Lmax.

5. Procédé selon l'une quelconque des revendications 1 à 4, dans lequel, à l'étape de mise en contact, une pluralité de cassures de jonction sont générées et chaque côté des cassures de jonction est marqué avec des codes-barres corrélés identifiant un côté en amont et en aval de la jonction.

6. Procédé selon l'une quelconque des revendications 1 à 5, comprenant en outre l'amplification des fragments d'acide nucléique.

7. Procédé selon l'une quelconque des revendications 1 à 6, dans lequel le pool de molécules d'acide nucléique comprend des molécules d'acide nucléique sans erreurs et contenant des erreurs ; dans lequel de préférence le procédé comprend en outre l'amplification des molécules d'acide nucléique sans erreurs possédant une séquence prédéterminée en utilisant des amorces possédant une séquence complémentaire à une séquence des marqueurs oligonucléotidiques d'extrémité 5' et d'extrémité 3' ; dans lequel, de manière davantage préférée, le procédé comprend en outre l'isolement des molécules d'acide nucléique sans erreurs possédant la séquence prédéterminée.

8. Procédé pour préparer des molécules d'acide nucléique, le procédé comprenant :
(a) la mise à disposition d'un pool de molécules d'acide nucléique synthétiques différentes possédant une longueur de 1 kbase ou plus, chaque molécule d'acide nucléique synthétique possédant une séquence d'acide nucléique cible unique, où chaque séquence d'acide nucléique cible possède une séquence de marqueur oligonucléotidique à l'extrémité 5' et à l'extrémité 3', et où la séquence de marqueur oligonucléotidique comprend un marqueur nucléotidique unique ;
(b) la soumission des molécules d'acide nucléique synthétiques à une fragmentation en utilisant des transpososomes afin de générer des fragments d'acide nucléique, où chaque fragment d'acide nucléique comprend une séquence de code-barres à l'extrémité 5' et à l'extrémité 3' ; et
(c) la détermination de la séquence des fragments d'acide nucléique marqués.

9. Procédé selon la revendication 8, dans lequel le pool de molécules d'acide nucléique synthétiques comprend des molécules d'acide nucléique sans erreurs et contenant des erreurs.

10. Procédé selon la revendication 8 ou 9, dans lequel, à l'étape de soumission, une pluralité de cassures de jonction sont générées et chaque côté des cassures de jonction est marqué avec des codes-barres oligonucléotidiques corrélés identifiant un côté en amont et en aval de la cassure de jonction.

11. Procédé pour préparer des molécules d'acide nucléique, le procédé comprenant :
(a) la mise à disposition d'un pool de molécules d'acide nucléique synthétiques possédant une longueur de 1 kbase ou plus comprenant au moins deux molécules d'acide nucléique différentes, le pool de molécules d'acide nucléique comprenant des molécules d'acide nucléique sans erreurs et contenant des erreurs, et où chaque population de molécule d'acide nucléique possède une séquence d'acide nucléique cible unique possédant une extrémité 5' et une extrémité 3' ;
(b) le marquage de l'extrémité 5' et de l'extrémité 3' des molécules d'acide nucléique cibles avec une séquence de marqueur oligonucléotidique, où la séquence de marqueur oligonucléotidique comprend un marqueur nucléotidique unique, en formant ainsi des molécules d'acide nucléique cibles marquées ;
(c) la dilution des molécules d'acide nucléique cibles marquées afin de générer un pool de molécules cibles marquées diluées comprenant au moins une molécule d'acide nucléique cible marquée sans erreurs ;
(d) la mise à disposition de transpososomes, où chaque transpososome possède un code-barres oligonucléotidique double brin unique différent ;
(e) l'ajout des transpososomes au pool de molécules d'acide nucléique marquées ;
(f) le fait de permettre aux transpososomes de générer des cassures de jonction d'acide nucléique double brin, en générant ainsi une pluralité de fragments d'acide nucléique comprenant un code-barres ou une séquence de marqueur oligonucléotidique à l'extrémité 5' et à l'extrémité 3' ; et
(g) la détermination de la séquence des fragments d'acide nucléique marqués.

12. Procédé selon la revendication 11, dans lequel, suite à l'étape de dilution, les molécules d'acide nucléique cibles marquées sont amplifiées.

13. Procédé selon la revendication 12, comprenant en outre la dilution des molécules d'acide nucléique cibles marquées amplifiées.

14. Procédé selon l'une quelconque des revendications 11 à 13, comprenant en outre l'amplification de la pluralité de fragments d'acide nucléique marqués.

15. Procédé selon l'une quelconque des revendications 11 à 14, comprenant en outre l'isolement des molécules d'acide nucléique sans erreurs possédant la séquence prédéterminée.


### SEQUENCE LISTING

<110> GEN9, INC.
<120> Methods for the Production of Long Length Clonal Sequence verified Nucleic Acid Constructs
<130> 127662-014302/PCT
<140> Not yet assigned
   <141> 2014-07-30
<150> 61/859,946
   <151> 2013-07-30
<150> 61/909,526
   <151> 2013-11-27
<160> 4
<170> PatentIn version 3.5
<210> 1
   <211> 30
   <212> DNA
   <213> Artificial Sequence
<220>
   <223> Synthetic construct
<400> 1
   cttattacga cgagtggctc atggtttttg 30
<210> 2
   <211> 30
   <212> DNA
   <213> Artificial Sequence
<220>
   <223> Synthetic construct
<400> 2
   cttattacga cgagtggctc atggtttttg 30
<210> 3
   <211> 30
   <212> DNA
   <213> Artificial Sequence
<220>
   <223> Synthetic construct
<400> 3
   cttattacga cgagtggctc atggtttttg 30
<210> 4
   <211> 29
   <212> DNA
   <213> Artificial Sequence
<220>
   <223> Synthetic Construct
<400> 4
   cttattacga cgagtggctc atgtttttg 29